# EUROPÄISCHE PATENTSCHRIFT

(11) **EP 2 391 596 B1**
(45) Veröffentlichungstag und Bekanntmachung des Hinweises auf die Patenterteilung: **20.06.2018**
(21) Anmeldenummer: 10701243.7
(22) Anmeldetag: 27.01.2010
(51) Int. Cl.: C07C 45/28, C07C 47/21, A61K 8/33, A61Q 13/00, C11B 9/00, C07C 45/62, C07C 45/82, C07C 47/02

(54) **VERFAHREN ZUR ISOLIERUNG VON DODECATRIENAL UND SEINE VERWENDUNG ALS AROMASTOFF**
METHOD FOR ISOLATING DODECATRIENAL AND USE THEREOF AS A FLAVOURING
PROCÉDÉ POUR ISOLER DU DODÉCATRIÉNAL ET SON UTILISATION EN TANT QUE PRODUIT AROMATIQUE

(30) Priorität: 28.01.2009 EP 09151526
(43) Veröffentlichungstag der Anmeldung: 07.12.2011
(73) Patentinhaber: BASF SE, 67056 Ludwigshafen am Rhein (DE)
(72) Erfinder: TELES, Joaquim, Henrique, 67166 Otterstadt (DE); ROESSLER-FEIGEL, Beatrice, 67256 Weisenheim am Sand (DE); HAUK, Alexander, 67067 Ludwigshafen (DE); MUELLER, Christian, 68167 Mannheim (DE); SCHELPER, Michael, 67056 Ludwigshafen (DE); KIRCHNER, Tanja, 55283 Nierstein (DE); SZESCHKUS, Susanne, 55232 Alzey (DE)
(86) Internationale Anmeldenummer: PCT/EP2010/050887
(87) Internationale Veröffentlichungsnummer: WO 2010/086313

(56) Entgegenhaltungen:
- WO-A1-2008/000757
- WO-A2-2005/030690
- DATABASE WPI Week 198709 Thomson Scientific, London, GB; AN 1987-059552 XP002588655 & JP 62 012735 A (KAO CORP) 21. Januar 1987 (1987-01-21)
- STAROKON E V ET AL: "LIQUID PHASE OXIDATION OF ALKENES WITH NITROUS OXIDE TO CARBONYL COMPOUNDS" ADVANCED SYNTHESIS & CATALYSIS, WILEY VCH VERLAG, WEINHEIM, DE LNKD- DOI:10.1002/ADSC.200303155, Bd. 346, Nr. 2-3, 1. Januar 2004 (2004-01-01), Seiten 268-274, XP002321384 ISSN: 1615-4150

## Beschreibung

Die vorliegende Erfindung betrifft ein Verfahren zur Herstellung einer Verbindung mit Z-1 Cyclen und 7 bis 16 C-Atomen mit mindestens einer Aldehydgruppe, umfassend die Stufen:
(a1) Oxidation eines cyclischen Olefins mit Z Cyclen und 7 bis 16 C-Atomen und mindestens zwei C-C-Doppelbindungen, ausgewählt aus der Gruppe bestehend aus 1,5-Cyclooctadien, 1,5-Cyclododecadien, 1,9-Cyclohexadecadien, 1,8-Cyclotetradecadien, 1,6-Cyclodecadien, 1,6,11-Cyclopentadecatrien, 1,5,9-Cyclododecatrien, Vinylcyclohexen, Norbornadien, Ethylidennorbornen, mittels Distickstoffmonoxid unter Erhalt einer Zusammensetzung (A1), mindestens enthaltend
   - eine cyclische Verbindung mit Z Cyclen und 7 bis 16 C-Atomen mit einer Ketogruppe,
   - ein cyclisches Olefin mit Z Cyclen und 7 bis 16 C-Atomen mit mindestens zwei C-C-Doppelbindungen und
   - eine Verbindung mit Z-1 Cyclen und 7 bis 16 C-Atomen mit mindestens einer Aldehydgruppe und mindestens zwei C-C-Doppelbindungen,
(a2) Abtrennen des cyclischen Olefins mit Z Cyclen und 7 bis 16 C-Atomen mit mindestens zwei C-C-Doppelbindungen aus der Zusammensetzung (A1) aus Stufe (a1), um eine Zusammensetzung (A2) zu erhalten, mindestens enthaltend
   - die cyclische Verbindung mit Z Cyclen und 7 bis 16 C-Atomen mit einer Ketogruppe und
   - die Verbindung mit Z-1 Cyclen und 7 bis 16 C-Atomen mit mindestens einer Aldehydgruppe und mindestens zwei C-C-Doppelbindungen,
(b1) Abtrennen der cyclischen Verbindung mit Z Cyclen und 7 bis 16 C-Atomen mit einer Ketogruppe aus der Zusammensetzung (A2) aus Schritt (a2), um eine Zusammensetzung (B1) zu erhalten, enthaltend mindestens 50 Gew.-% der Verbindung mit Z-1 Cyclen und 7 bis 16 C-Atomen mit mindestens einer Aldehydgruppe und mindestens zwei C-C-Doppelbindungen,
(b2) Reinigung der in Schritt (b1) erhaltenen Zusammensetzung (B1), um eine Mischung zu erhalten, die mindestens 92 Gew.-% der Verbindung mit Z-1 Cyclen und 7 bis 16 C-Atomen mit mindestens einer Aldehydgruppe und mindestens zwei C-C-Doppelbindungen enthält,
wobei Z 1, 2, 3 oder 4 sein kann, und Stufe (b1) in mindestens zwei Destillationskolonnen T1 und T2, wobei T1 mindestens 15 theoretische Trennstufen und T2 mindestens 30 Trennstufen aufweisen, oder in einer Trennwandkolonne, durchgeführt wird.

Des Weiteren betrifft die vorliegende Erfindung die Verwendung einer Mischung, enthaltend 92 bis 99,8 Gew.-% mindestens eines 4,8,11-Dodecatrienals und 0,0001 bis 5 Gew.-% 1,5,9-Cyclododecatriene, als Riechstoff.

Die Verwendung von Verbindungen, die mindestens eine Aldehydgruppe und mindestens zwei C-C-Doppelbindungen aufweisen, als Riechstoffe bzw. Verfahren zur Herstellung dieser Verbindungen sind aus dem Stand der Technik bereits bekannt. Die einzigen kommerziellen Aldehyde mit mindestens zwei C-C-Doppelbindungen die als Riechstoffe eingesetzt werden, sind in der nächsten Abbildung angegeben, wobei in eckigen Klammern der Handelsname angegeben wird (siehe hierzu H. Surburg, J. Panten, "Common Fragrance and Flavor Materials", 5th ed., Wiley-VCH (2006)).

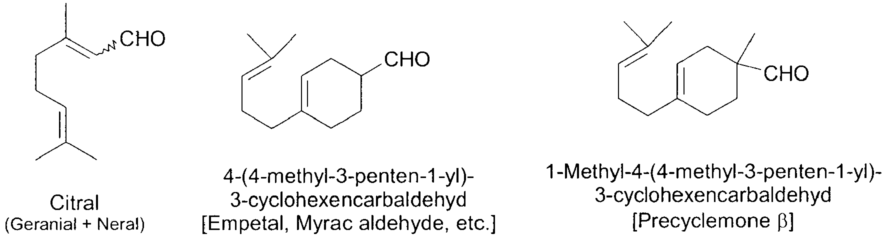

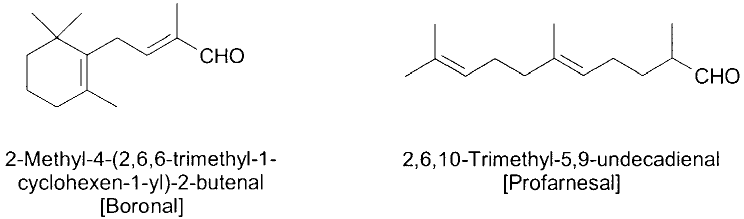

In den japanischen Patentanmeldungen mit den Offenlegungsnummern S62-16412 und S62-12735 wird ein Verfahren zur Herstellung von 2E,4E-(oder 4Z)-2,4,11-Dodecatrienal offenbart, in dem 2E-(oder 2Z)-Decadienal mit dem entsprechenden Wittig-Reagenz unter Erhalt des Diethylvollacetals des gewünschten Produktes umgesetzt wird. Saure Hydrolyse des Vollacetals liefert den entsprechenden Aldehyd.

Blank et al., J. Agric. Food Chem. 2001, 49, 2959-2965 offenbaren ein Verfahren zur Herstellung von (E,Z,Z)-2,4,7-Tridecatrienal durch Oxidation von Arachidonsäure, und die Verwendung dieses ungesättigten Aldehyds als Duftstoff.

Ran et al., Tetrahedron Letters 45 (2004), 7851-7853, offenbaren ein Verfahren zur Herstellung von 2E,4E,6E-Dodecatrienal. Dieses Verfahren basiert auf einer regioselektiven Kettenverlängerung von 2E,4E-Decadienal.

Bohlmann et al., Liebigs Ann. Chem. 1982, Seiten 1216 bis 1218, offenbaren ein Verfahren zur Herstellung von (2E,4Z)-2,4,11-Dodecatrien-1-al durch Umsetzung des entsprechenden Phosphorylids basierend auf 1,7-Octadien mit Fumaraldehydsäureethylester und anschließender Reduktion der Esterfunktion zur Aldehydfunktion.

WO 2008/000757 A1 offenbart ein Verfahren zur Herstellung eines cyclischen Ketons mit 7 bis 16 C-Atomen durch Oxidation mindestens eines cyclischen Olefins mit 7 bis 16 C-Atomen mit mindestens einer C-C-Doppelbindung mittels Distickstoffmonoxid und Behandeln der aus der Oxidation erhaltenen Zusammensetzung mit einer Base. Dabei wird aus der Oxidation eine Zusammensetzung (E) erhalten, die Cyclododeca-4,8-dienon als Hauptprodukt und Dodeca-4,8,11-trienal und weitere Verbindungen als Nebenprodukte enthält. Aus diesem Gemisch wird zunächst nicht umgesetztes Edukt, Cyclododecatrien, abgetrennt und Dodeca-4,8,11-trienal durch Destillation abgereichert.

WO 2005/030690 A2 offenbart ein Verfahren zur Herstellung eines Ketons, insbesondere von Cyclododecanon, wobei Cyclododecatrien mit Distickstoffmonoxid und der Erhalt von Cyclododecadienon umgesetzt wird und das erhaltene Cyclododecadienon insbesondere zu Cyclododecanon hydriert wird. Bei der Umsetzung von Cyclododecatrien mit Di-Stickstoffmonoxid wird im Allgemeinen ein Gemisch erhalten, welches Cyclododecadienon und gegebenenfalls nicht umgesetztes Edukt und/oder gegebenenfalls mindestens ein Nebenprodukt enthält. In den Beispielen 1, 2 und 3 wird offenbart, dass bei der Oxidation von 1,5,9-Cyclododecatrien mit Distickstoffmonoxid Dodeca-4,8,11-trienal als Nebenprodukt entsteht.

JP 62 012 735 A offenbart (2Z, 4E (oder 4Z))-2,4,11-Dodecatrienal und Parfum-Zusammensetzungen, die dieses enthalten.

E. V. Starokon et al., Adv. Synth. Catal. 2004, 346, 268-274 offenbart allgemein, dass Alkene mit Distickstoffmonoxid oxidiert werden können.

4,8,11-Dodecatrienale sind durch eine gezielte Synthese nur schwer darstellbar, und bisher ist in der Literatur keine Synthese beschrieben. Lineare ungesättigte Aldehyde sind bekannt als wertvolle Riechstoffe (H. Surburg, J. Panten, "Common Fragrance and Flavor Materials" Wiley-VCH, 5th edition, 2006, Seiten 15 und 16).

Eine Aufgabe der vorliegenden Erfindung ist es daher, ein Verfahren bereitzustellen, mit dem cyclische oder acyclische Verbindungen mit 7 bis 16 C-Atomen mit mindestens einer Aldehydgruppe und mindestens zwei C-C-Doppelbindungen in guter Ausbeute und hoher Reinheit erhalten werden können. Eine weitere Aufgabe der vorliegenden Erfindung ist es, dass bereits bestehende Anlagen bzw. Apparaturen zur Herstellung von cyclischen Ketonen mit 7 bis 16 C-Atomen weiter genutzt werden können, um den zusätzlichen apparativen Aufbau möglichst gering zu halten.

Diese Aufgaben werden erfindungsgemäß gelöst durch ein Verfahren zur Herstellung einer Verbindung mit Z-1 Cyclen und 7 bis 16 C-Atomen mit mindestens einer Aldehydgruppe, umfassend die Stufen:
(a1) Oxidation eines cyclischen Olefins mit Z Cyclen und 7 bis 16 C-Atomen und mindestens zwei C-C-Doppelbindungen, ausgewählt aus der Gruppe bestehend aus 1,5-Cyclooctadien, 1,5-Cyclododecadien, 1,9-Cyclohexadecadien, 1,8-Cyclotetradecadien, 1,6-Cyclodecadien, 1,6,11-Cyclopentadecatrien, 1,5,9-Cyclododecatrien, Vinylcyclohexen, Norbornadien, Ethylidennorbornen, mittels Distickstoffmonoxid unter Erhalt einer Zusammensetzung (A1), mindestens enthaltend
   - eine cyclische Verbindung mit Z Cyclen und 7 bis 16 C-Atomen mit einer Ketogruppe,
   - ein cyclisches Olefin mit Z Cyclen und 7 bis 16 C-Atomen mit mindestens zwei C-C-Doppelbindungen und
   - eine Verbindung mit Z-1 Cyclen und 7 bis 16 C-Atomen mit mindestens einer Aldehydgruppe und mindestens zwei C-C-Doppelbindungen,
(a2) Abtrennen des cyclischen Olefins mit Z Cyclen und 7 bis 16 C-Atomen mit mindestens zwei C-C-Doppelbindungen aus der Zusammensetzung (A1) aus Stufe (a1), um eine Zusammensetzung (A2) zu erhalten, mindestens enthaltend
   - die cyclische Verbindung mit Z Cyclen und 7 bis 16 C-Atomen mit einer Ketogruppe und
   - die Verbindung mit Z-1 Cyclen und 7 bis 16 C-Atomen mit mindestens einer Aldehydgruppe und mindestens zwei C-C-Doppelbindungen,
(b1) Abtrennen der cyclischen Verbindung mit Z Cyclen und 7 bis 16 C-Atomen mit einer Ketogruppe aus der Zusammensetzung (A2) aus Schritt (a2), um eine Zusammensetzung (B1) zu erhalten, enthaltend mindestens 50 Gew.-% der Verbindung mit Z-1 Cyclen und 7 bis 16 C-Atomen mit mindestens einer Aldehydgruppe und mindestens zwei C-C-Doppelbindungen,
(b2) Reinigung der in Schritt (b1) erhaltenen Zusammensetzung (B1), um eine Mischung zu erhalten, die mindestens 92 Gew.-% der Verbindung mit Z-1 Cyclen und 7 bis 16 C-Atomen mit mindestens einer Aldehydgruppe und mindestens zwei C-C-Doppelbindungen enthält,
wobei Z 1, 2, 3 oder 4 sein kann, und Stufe (b1) in mindestens zwei Destillationskolonnen T1 und T2, wobei T1 mindestens 15 theoretische Trennstufen und T2 mindestens 30 Trennstufen aufweisen, oder in einer Trennwandkolonne, durchgeführt wird.

Es wurde überraschend gefunden, dass insbesondere Aldehyde, die einen Cyclus weniger (Z-1 Cyclen) aufweisen als die eingesetzten Ausgangsverbindungen, durch Oxidation der genannten Edukte mit Distickstoffmonoxid in einer hohen Reinheit und guter Ausbeute zu erhalten. In einer bevorzugten Ausführungsform weist die durch das erfindungsgemäße Verfahren herstellbare Verbindung mit Z-1 Cyclen und 7 bis 16 C-Atomen mit mindestens einer Aldehydgruppe eine Aldehydgruppe auf.

Durch das erfindungsgemäße Verfahren können insbesondere Verbindungen mit Z-1 Cyclen und 7 bis 16 C-Atomen und mindestens einer Aldehydgruppe und mindestens zwei C-C-Doppelbindungen mit einer Reinheit von beispielsweise mindestens 95 %, bevorzugt mindestens 98 %, erhalten werden. Die Reinheit kann nach allen dem Fachmann bekannten Verfahren bestimmt werden, beispielsweise Gaschromatographie. Ein weiterer Vorteil des erfindungsgemäßen Verfahrens ist, dass es leicht mit bestehenden Anlagen kombiniert werden kann, so dass keine kostenintensiven Umbauten erforderlich sind.

Im Folgenden werden die einzelnen Stufen des erfindungsgemäßen Verfahrens detailliert beschreiben:

### Stufe (a1)

Die Umsetzung gemäß Stufe (a1) kann generell gemäß sämtlichen Verfahrensführungen erfolgen, bei denen das Olefin und Distickstoffmonoxid miteinander reagieren.

In Stufe (a1) des erfindungsgemäßen Verfahrens wird das cyclische Olefin durch Umsetzung mit Distickstoffmonoxid oxidiert. Dabei kann für die Umsetzung des cyclischen Olefins mit Distickstoffmonoxid mindestens ein geeignetes Lösungsmittel oder Verdünnungsmittel eingesetzt werden. Als solche sind unter anderem cyclische Alkane, beispielsweise Cyclododecan oder Cyclododecanon oder gesättigte aliphatische oder aromatische, gegebenenfalls alkylsubstituierte Kohlenwasserstoffe zu nennen, wobei im Wesentlichen alle gängigen Lösungs- und/oder Verdünnungsmittel geeignet sind unter der Maßgabe, dass sie weder eine C-C-Doppelbindung, noch eine C-C-Dreifachbindung, noch eine Aldehydgruppe aufweisen.

Im Allgemeinen ist bei der Umsetzung des cyclischen Olefins mit Distickstoffmonoxid der Zusatz eines Lösungsmittels oder Verdünnungsmittels nicht notwendig.

Die Temperatur bei der Umsetzung des cyclischen Olefins mit Distickstoffmonoxid liegt bevorzugt bei 140 bis 350 °C, weiter bevorzugt bei 180 bis 320 °C und besonders bevorzugt bei 200 bis 300 °C.

Es ist möglich, die Umsetzung des cyclischen Olefins mit Distickstoffmonoxid bei zwei oder mehr Temperaturen beziehungsweise in zwei oder mehr Temperaturbereichen durchzuführen, die jeweils in den oben angegebenen Grenzen liegen. Temperaturänderungen im Laufe der Umsetzung können kontinuierlich oder auch diskontinuierlich vollzogen werden.

Der Druck bei der Umsetzung des cyclischen Olefins mit Distickstoffmonoxid liegt bevorzugt höher als der Eigendruck des Edukt- bzw. Produktgemisches bei der gewählten Reaktionstemperatur oder den gewählten Reaktionstemperaturen. Der Druck liegt bevorzugt bei 1 bis 1000 bar, weiter bevorzugt bei 40 bis 325 bar und besonders bevorzugt bei 50 bis 200 bar.

Es ist möglich, die Umsetzung des cyclischen Olefins mit Distickstoffmonoxid bei zwei oder mehr Drücken beziehungsweise in zwei oder mehr Druckbereichen durchzuführen, die jeweils in den oben angegebenen Grenzen liegen. Druckänderungen im Laufe der Umsetzung können kontinuierlich oder auch diskontinuierlich vollzogen werden.

Hinsichtlich der für die Umsetzung des cyclischen Olefins mit Distickstoffmonoxid einsetzbaren Reaktoren existieren keine besonderen Beschränkungen. Insbesondere kann die Umsetzung in Batch-Fahrweise oder in kontinuierlicher Fahrweise erfolgen. Demgemäß können beispielsweise als Reaktoren mindestens ein CSTR (Continuous Stirred Tank Reactor) mit mindestens einem internen und/oder mindestens einem externen Wärmetauscher, mindestens ein Rohrreaktor, mindestens ein Rohrbündelreaktor oder mindestens ein Schlaufenreaktor eingesetzt werden. Ebenso ist es möglich, mindestens einen dieser Reaktoren so auszugestalten, dass er mindestens zwei unterschiedliche Zonen aufweist. Solche Zonen können sich beispielsweise in Reaktionsbedingungen wie beispielsweise der Temperatur oder dem Druck und/oder in der Geometrie der Zone wie beispielsweise dem Volumen oder dem Querschnitt unterscheiden. Wird die Umsetzung in zwei oder mehr Reaktoren durchgeführt, können zwei oder mehr gleiche Reaktortypen oder mindestens zwei verschiedene Reaktortypen eingesetzt werden.

Bevorzugt wird die Umsetzung des cyclischen Olefins mit Distickstoffmonoxid in einem einzigen Reaktor durchgeführt. Beispielsweise bevorzugt ist die Umsetzung in kontinuierlicher Fahrweise. Ein geeigneter Reaktor ist beispielsweise in der noch nicht veröffentlichten Patentanmeldung EP 09151002.4 beschrieben.

Die Verweilzeit des Reaktionsgutes in dem mindestens einen Reaktor bei der Umsetzung des cyclischen Olefins mit Distickstoffmonoxid liegt im allgemeinen im Bereich von bis zu 20 Stunden, bevorzugt im Bereich von 0,1 bis 20 Stunden, weiter bevorzugt im Bereich von 0,2 bis 15 Stunden und besonders bevorzugt im Bereich von 0,25 bis 10 Stunden.

Im Feed, der der Umsetzung von Distickstoffmonoxid mit dem cyclischen Olefin zugeführt wird, liegt das Molverhältnis von Distickstoffmonoxid und dem cyclischen Olefin im Allgemeinen im Bereich von 0,05 bis 4, bevorzugt im Bereich von 0,06 bis 1, weiter bevorzugt im Bereich von 0,07 bis 0,5 und besonders bevorzugt im Bereich von 0,1 bis 0,4.

Die Umsetzung des cyclischen Olefins mit Z Cyclen und 7 bis 16 C-Atomen mit mindestens zwei C-C-Doppelbindungen mit Distickstoffmonoxid kann so durchgeführt werden, dass ein Umsatz des cyclischen Olefins im Bereich von bis zu 50%, bevorzugt im Bereich von 5 bis 30% und insbesondere bevorzugt im Bereich von 10 bis 20% erreicht wird.

Grundsätzlich kann erfindungsgemäß jedes cyclische Olefin mit Z Cyclen und 7 bis 16 C-Atomen mit mindestens zwei C-C-Doppelbindungen oder jedes Gemisch aus zwei und mehr unterschiedlichen cyclischen Olefinen mit Z Cyclen 7 bis 16 C-Atomen mit jeweils mindestens zwei C-C-Doppelbindungen mit Distickstoffmonoxid umgesetzt werden.

Erfindungsgemäß bedeutet die Formulierung "Z Cyclen", dass die entsprechend beschriebenen Verbindungen cyclische Einheiten in einer Anzahl von Z aufweisen. Erfindungsgemäß bedeutet Z 1, 2, 3 oder 4, beispielsweise ist für die bevorzugten Verbindungen (I) bis (VIII) und (XI) Z gleich 1, für die bevorzugten Verbindungen (IX) und (X) ist Z gleich 2. Für den besonders bevorzugten Fall, dass Z gleich 1 ist, werden somit durch das erfindungsgemäße Verfahren besonders bevorzugt acyclische Verbindungen (Z = 1, daraus folgt Z-1 = 0), d. h. Verbindungen, die keinen Cyclus aufweisen, mit 7 bis 16 C-Atomen und mindestens einer Aldehydgruppe und mindestens zwei C-C-Doppelbindungen hergestellt.

Bevorzugt weist das cyclische Olefin erfindungsgemäß zwei, drei oder vier C-C-Doppelbindungen auf.

Daher betrifft die vorliegende Erfindung gemäß einer weiteren Ausführungsform auch ein wie zuvor beschriebenes Verfahren zur Herstellung einer Verbindung mit Z-1 Cyclen und 7 bis 16 C-Atomen mit mindestens einer Aldehydgruppe, wobei das cyclische Olefin drei C-C-Doppelbindungen aufweist.

Die vorliegende Erfindung betrifft das erfindungsgemäße Verfahren wobei das cyclische Olefin mit mindestens zwei C-C-Doppel-bindungen ausgewählt ist aus der Gruppe bestehend aus 1,5-Cyclooctadien, 1,5-Cyclododecadien, 1,9-Cyclohexadecadien, 1,8-Cyclotetradecadien, 1,6-Cyclodecadien, 1,6,11-Cyclopentadecatrien, 1,5,9-Cyclododecatrien, Vinylcyclohexen, Norbornadien, Ethylidennorbornen. 1,5-Cyclooctadien, 1,5-Cyclododecadien, 1,9-Cyclohexadecadien, 1,8-Cyclotetradecadien, 1,6-Cyclodecadien, 1,6,11-Cyclopentadecatrien, 1,5,9-Cyclododecatrien und Vinylcyclohexen weisen einen Cyclus auf, somit ist in diesen Fällen Z gleich eins. Bei Norbornadien und Ethylidennorbornen ist Z gleich zwei.

1,5-Cyclooctadien (I), 1,5-Cyclododecadien (II), 1,9-Cyclohexadecadien (III), 1,8-Cyclotetradecadien (IV), 1,6-Cyclododecadien (V), 1,6,11-Cyclopentadecatrien (VI), 1,5,9-Cyclododecatrien (VII), 1,5,9,13-Cyclohexadecatetraen (VII), Norbornadien (IX), Ethylidennorbornen (X), Vinylcyclohexen (XI), wobei immer nur eines der möglichen Isomere dargestellt ist, sind im Folgenden abgebildet:

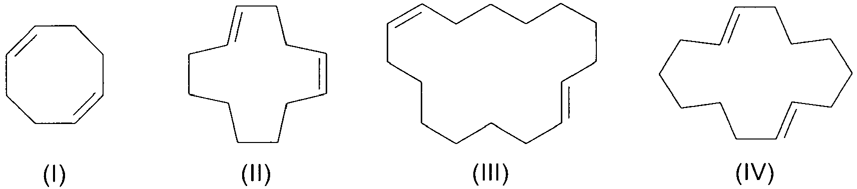

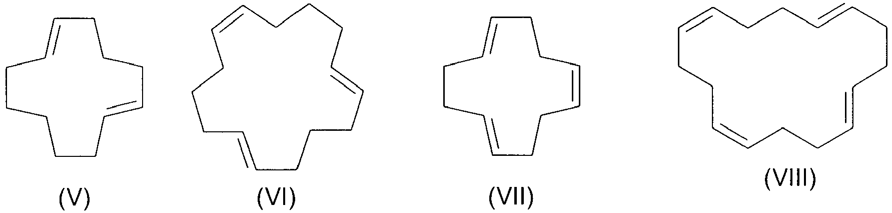

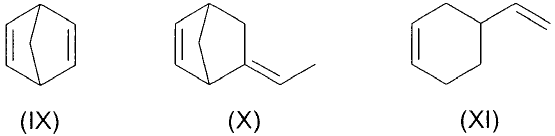

Besonders bevorzugt wird als cyclisches Olefin 1,5,9-Cyclododecatrien (VII) eingesetzt. 1,5,9-Cyclododecatrien kann im Allgemeinen in jedem möglichen Isomer eingesetzt werden, beispielsweise cis,trans,trans-1,5,9-Cyclododecatrien, cis,cis,trans-1,5,9-Cyclododecatrien, all-trans-1,5,9-Cyclododecatrien oder all-cis-1,5,9-Cyclododecatrien, ganz besonders bevorzugt cis,trans,trans-1,5,9-Cyclododecatrien. In dem erfindungsgemäßen Verfahren kann auch eine Mischung der genannten Isomere umgesetzt werden und insbesondere ein Isomerengemisch, das überwiegend cis,trans,trans-1,5,9-Cyclododecatrien enthält.

Daher betrifft die vorliegende Erfindung in einer bevorzugten Ausführungsform ein Verfahren zur Herstellung eines Aldehyds wie oben beschrieben, wobei das cyclische Olefin mit Z Cyclen und 7 bis 16 C-Atomen mit mindestens zwei Doppelbindungen Cyclododecatrien, bevorzugt 1,5,9-Cyclododecatrien, besonders bevorzugt cis,trans,trans-1,5,9-Cyclododecatrien, ist.

Das in dem erfindungsgemäßen Verfahren vorzugsweise eingesetzte Cyclododecatrien kann im Allgemeinen durch alle dem Fachmann bekannte Verfahren erhalten werden, In einer bevorzugten Ausführungsform wird das Cyclododecatrien durch Trimerisierung von Butadien erhalten.

Daher betrifft die vorliegende Erfindung gemäß einer weiteren Ausführungsform auch ein wie zuvor beschriebenes Verfahren zur Herstellung einer Verbindung mit Z Cyclen und 7 bis 16 C-Atomen mit mindestens einer Aldehydgruppe und mindestens zwei C-C-Doppelbindungen, wobei das cyclische Olefin mit Z Cyclen und 7 bis 16 C-Atomen mit mindestens zwei Doppelbindungen 1,5,9-Cyclododecatrien ist, das mittels Trimerisierung aus Butadien hergestellt wurde.

1,5,9-Cyclododecatrien kann beispielsweise durch Trimerisierung von reinem 1,3-Butadien hergestellt werden, wie dies beispielsweise in T. Schiffer, G. Oenbrink, "Cyc-Iododecatriene, Cyclooctadiene, and 4-Vinylcyclohexene", in Ullmann's Encyclopedia of Industrial Chemistry, 6th Edition (2000), Electronic Release, Wiley VCH, Seiten 1 bis 4, beschrieben ist. Im Rahmen dieses Verfahrens entstehen beispielsweise bei der Trimerisierung in Anwesenheit von Ziegler-Katalysatoren cis,trans,trans-1,5,9-Cyclododecatrien, cis,cis,trans-1,5,9-Cyclododecatrien und all-trans-1,5,9-Cyclododecatrien, wie dies beispielsweise in H. Weber et al. "Zur Bildungsweise von cis,trans,trans-Cyclododecatrien-(1.5.9) mittels titanhaltiger Katalysatoren" in Liebigs Ann. Chem. 681 (1965) Seiten 10 bis 20 beschrieben ist. Cyclododecatrien kann beispielsweise durch Trimerisierung von 1,3-Butadien unter Verwendung eines Titan- oder NickelKatalysators, beispielsweise gemäß DE 1283836, hergestellt werden.

Während grundsätzlich sämtliche geeigneten Titan-Katalysatoren zur Trimerisierung eingesetzt werden können, ist der im Artikel von Weber et al. beschriebene Titantetrachlorid/Ethylaluminiumsesquichlorid-Katalysator besonders geeignet.

Während grundsätzlich sämtliche geeigneten Nickel-Katalysatoren zur Trimerisierung eingesetzt werden können, ist der in der DE 1283836 beschriebene bis-Cyclooctadienylnickel/Ethoxydiethylaluminium-Katalysator besonders geeignet.

Das für die Trimerisierung eingesetzte Butadien weist insbesondere bevorzugt einen über Gaschromatographie bestimmten Reinheitsgrad von mindestens 99,6% und weiter bevorzugt von mindestens 99,65% auf. Insbesondere bevorzugt enthält das eingesetzte 1,3-Butadien im Rahmen der Nachweisgenauigkeit kein 1,2-Butadien und kein 2-Butin.

Aus dieser Trimerisierung werden im Allgemeinen Gemische erhalten, die mindestens 95 Gew.-%, bevorzugt mindestens 96 Gew.-% und weiter bevorzugt mindestens 97 Gew.-% cis,trans,trans-1,5,9-Cyclododecatrien enthalten. Insbesondere bevorzugt enthalten die Gemische in etwa 98 Gew.-% cis,trans,trans-1,5,9-Cyclododecatrien.

Dieses cis,trans,trans-1,5,9-Cyclododecatrien enthaltende Gemisch kann als solches für die erfindungsgemäße Umsetzung gemäß Stufe (a1) verwendet werden. Ebenso ist es möglich, über mindestens eine geeignete Methode, beispielsweise bevorzugt über mindestens eine Destillation, das cis,trans,trans-1,5,9-Cyclododecatrien aus dem Gemisch abzutrennen und in der Umsetzung gemäß Stufe (a1) einzusetzen.

Gemäß einer ganz besonders bevorzugten Ausführungsform des erfindungsgemäßen Verfahrens wird als Cyclododecatrien ein Isomerengemisch eingesetzt, das überwiegend cis,trans,trans-1,5,9-Cyclododecatrien, trans,trans,trans-1,5,9-Cyclododecatrien oder cis,cis,trans-1,5,9-Cyclododecatrien enthält. Vorzugsweise wird ein Isomerengemisch eingesetzt, das mehr als 60 Gew.-%, bezogen auf das Isomerengemisch, cis,trans,trans-1,5,9-Cyclododecatrien enthält, weiter bevorzugt mehr als 70 Gew.-%, insbesondere mehr als 80 Gew.-%, besonders bevorzugt mehr als 90 Gew.-%, beispielsweise mehr als 91 Gew.-%, mehr als 92 Gew.-%, mehr als 93 Gew.-%, mehr als 94 Gew.-%, mehr als 95 Gew.-%, mehr als 96 Gew.-%, mehr als 97 Gew.-% oder mehr als 98 Gew.-%.

Die erfindungsgemäß einsetzbaren Olefine können beispielsweise durch die in den folgenden Literaturstellen genannten Verfahren hergestellt werden:
(I) Cycloocta-1,5-dien fällt als Nebenprodukt bei der Synthese von Verbindung (VII) an, wie dies beispielsweise in T. Schiffer, G. Oenbrink, "Cyclododecatriene, Cyc-Iooctadiene, and 4-Vinylcyclohexene", in Ullmann's Encyclopedia of Industrial Chemistry, 6th Edition (2000), Electronic Release, Wiley VCH beschrieben ist.
(II) Cyclododeca-1,5-dien kann zum Beispiel durch katalytische Reduktion von Verbindung (VII) gewonnen werden, wie zum Beispiel in der US 3,182,093 beschrieben wird.
(III) Cyclohexadeca-1,9-dien kann durch Metathese von Cycloocten gewonnen werden, wie zum Beispiel in EP 1288181 beschrieben wird.
(IV) Cyclotetradeca-1,8-dien kann durch Metathese von Cyclohepten gewonnen werden, wie zum Beispiel in S. Warwel, H. Kaetker, Synthesis (1987) (10), 935-7 beschrieben wird.
(V) Cyclodeca-1,6-dien, bevorzugt das cis,cis-Isomer, kann durch Isomerisierung von cis,trans-Cyclodeca-1,5-dien gewonnen werden, wie zum Beispiel in der DE 1 230 023 beschrieben wird.
(VI) Cyclopentadecadeca-1,6,11-trien kann durch Cyclooligomerisierung von Cyclopenten gewonnen werden, wie zum Beispiel in der DD 115480 beschrieben wird.
(VII) Siehe (I)
(VIII) Cyclohexadeca-1,5,9,13-tetraen kann durch Tetramerisierung von Butadien gewonnen werden, wie zum Beispiel in U. M. Dzhemilev, L. Yu. Gubaidullin, G. A. Tolstikov, Zhurnal Organicheskoi Khimii (1976), 12(1), 44-6 beschrieben wird.
(IX) Norbornadien kann durch Umsetzung von Cyclopentadien mit Acetylen gewonnen werden, wie zum Beispiel in der US 2875256 beschrieben wird.
(X) Ethylidennorbornen kann durch basenkatalysierte Umlagerung von 5-Vinyl-2-norbornen gewonnen werden, wie zum Beispiel in der EP 0 279 397 beschrieben wird.
(XI) 4-Vinylcyclohexen kann durch Diels-Alder-Reaktion von Butadien mit sich selbst hergestellt werden, fällt aber auch als Nebenprodukt bei der Herstellung von Verbindung (VII) an, wie zum Beispiel in T. Schiffer, G. Oenbrink, "Cyclododecatriene, Cyclooctadiene, and 4-Vinylcyclohexene", in Ullmann's Encyclopedia of Industrial Chemistry, 6th Edition (2000), Electronic Release, Wiley VCH, Seiten 1-4, beschrieben ist.

Im Allgemeinen resultiert aus der bevorzugten erfindungsgemäßen Umsetzung von cis,trans,trans-1,5,9-Cyclododecatrien mit Distickstoffmonoxid gemäß Stufe (a1) ein Cyclododeca-4,8-dienon-Isomerengemisch, das mindestens zwei der Isomere cis,trans-Cyclododeca-4,8-dienon, trans,cis-Cyclododeca-4,8-dienon und trans,trans-Cyclododeca-4,8-dienon als cyclische Verbindungen mit Z Cyclen und 7 bis 16 C-Atomen mit einer Ketogruppe enthält.

Bevorzugt wird erfindungsgemäß ein Isomerengemisch erhalten, bei dem trans,cis- und cis,trans-Isomere dieses Ketons in etwa gleichen Mengen gebildet werden, und das trans,trans-Isomer im Vergleich zu den beiden anderen Isomeren in nur geringen Mengen gebildet wird. Ein beispielsweise typisches Isomerengemisch weist demgemäß die genannten Isomeren in molaren Verhältnissen von in etwa 1 : 1 : 0,08 auf.

Die in Zusammensetzung (A1) enthaltene eine cyclische Verbindung mit Z Cyclen und 7 bis 16 C-Atomen mit einer Ketogruppe ist mengenmäßig das Hauptprodukt von Stufe (a1) des erfindungsgemäßen Verfahrens.

Da bevorzugt als Substrat eine cyclische Verbindung mit Z Cyclen und 7 bis 16 C-Atomen und drei Doppelbindungen eingesetzt wird, wird mengenmäßig als bevorzugtes Produkt in Stufe (a1) des erfindungsgemäßen Verfahrens durch Oxidation einer dieser Doppelbindungen mindestens eine cyclische Verbindung mit Z Cyclen und 7 bis 16 C-Atomen mit zwei Doppelbindungen und einer Ketogruppe gebildet. In einer bevorzugten Ausführungsform des erfindungsgemäßen Verfahrens wird diese cyclische Verbindung mit Z Cyclen und 7 bis 16 C-Atomen und einer Ketogruppe in einer noch folgenden Stufe zu mindestens einer gesättigten cyclischen Verbindung mit Z Cyclen und 7 bis 16 C-Atomen und einer Ketogruppe, insbesondere Cyclododecanon, hydriert.

Die Zusammensetzung (A1) enthält im Rahmen der vorliegenden Erfindung mindestens eine cyclische Verbindung mit Z Cyclen und 7 bis 16 C-Atomen mit einer Ketogruppe in einer Menge von im Allgemeinen mehr als 5 Gew.-%, bevorzugt mehr als 10 Gew.-%, vorzugsweise 10 bis 90 Gew.-%, insbesondere 11 bis 50 Gew.-%, besonders bevorzugt 12 bis 40 Gew.-%, insbesondere bevorzugt 13 bis 30 Gew.-%, beispielsweise 14 bis 20 Gew.-% oder 15 bis 18 Gew.-%.

Erfindungsgemäß liegen in Zusammensetzung (A1) auch mindestens
- das mindestens eine cyclische Olefin mit Z Cyclen und 7 bis 16 C-Atomen mit mindestens zwei C-C-Doppelbindungen und
- die mindestens eine Verbindung mit Z-1 Cyclen und 7 bis 16 C-Atomen mit mindestens einer Aldehydgruppe und mindestens zwei C-C-Doppelbindungen vor.

Das mindestens eine cyclische Olefin mit Z Cyclen und 7 bis 16 C-Atomen mit mindestens zwei C-C-Doppelbindungen, die in Zusammensetzung (A1) vorliegt, ist die gleiche Verbindung, die in Zusammensetzung (A) vor der Oxidation als Ausgangsverbindung eingesetzt worden ist. Das mindestens eine cyclische Olefin mit Z Cyclen und 7 bis 16 C-Atomen mit mindestens zwei C-C-Doppelbindungen in Zusammensetzung (A1) ist somit verbliebenes Edukt, welches in Stufe (a1) des erfindungsgemäßen Verfahrens nicht oxidiert worden ist. Erfindungsgemäß kann das mindestens eine cyclische Olefin mit Z Cyclen und 7 bis 16 C-Atomen mit mindestens zwei Doppelbindungen in Zusammensetzung (A1) in den gleichen isomeren Strukturen vorliegen, in denen es als Edukt eingesetzt worden ist. In einer bevorzugten Ausführungsform liegt in Zusammensetzung (A1) bezüglich des mindestens einen Olefins mit Z Cyclen und 7 bis 16 C-Atomen mit mindestens zwei C-C-Doppelbindungen ein etwas anderes Isomerenverhältnis vor, als in dem in Schritt (a1) eingesetzten Edukt. Dies ist beispielsweise in den verschiedenen Reaktivitäten der einzelnen Isomeren begründet, beispielsweise reagiert das all-trans-Isomer schneller als das cis,trans,trans-Isomer, und dieses wiederum etwas schneller als das cis,cis,trans-Isomer.

Die in der Zusammensetzung (A1) vorliegende gewünschte Produkt des erfindungsgemäßen Verfahrens, die mindestens eine Verbindung mit Z-1 Cyclen und 7 bis 16 C-Atomen mit mindestens einer Aldehydgruppe, entsteht im Allgemeinen aus dem als Edukt eingesetzten Olefin mit mindestens zwei Doppelbindungen durch oxidative Spaltung einer Doppelbindung.

Da, wie oben beschrieben, Z die Anzahl der in den genannten Verbindungen vorliegenden Cyclen beschreibt, bedeutet die Formulierung "Z-1 Cyclen", dass in dem gewünschten Produkt des erfindungsgemäßen Verfahrens ein Cyclus weniger vorliegt, als in den Verbindungen, die Z Cyclen enthalten. Das gewünschte Produkt wird erfindungsgemäß durch eine Ringöffnungsreaktion erhalten und weist daher einen Cyclus weniger als das Edukt auf.

In Stufe (a1) des erfindungsgemäßen Verfahrens wird gegebenenfalls auch mindestens eine Verbindung mit Z Cyclen und 7 bis 16 C-Atomen mit mindestens einer Aldehydgruppe als Nebenprodukt gebildet. Dieses mindestens eine Nebenprodukt weist die gleiche Anzahl an Cyclen wie das in Stufe (a1) eingesetzte Edukt auf. Bei dieser mindestens einen Verbindung handelt es sich bevorzugt um eine cyclische Verbindung mit mindestens einer Aldehydgruppe die durch Ringverengung entsteht. Dieses gegebenenfalls vorliegende Nebenprodukt weist somit die gleiche Anzahl an Cyclen auf, wie das Edukt.

Für den bevorzugten Fall, dass in dem erfindungsgemäßen Verfahren als Edukt 1,5,9-Cyclododecatrien, insbesondere cis,trans,trans-1,5,9-Cyclododecatrien (Z=1), eingesetzt wird, wird als das erfindungsgemäß gewünschte Produkt mindestens eine Verbindung mit Z-1 Cyclen und 7 bis 16 C-Atomen mit mindestens einer Aldehydgruppe und mindestens zwei C-C-Doppelbindungen besonders bevorzugt ein Isomerengemisch von acyclischen Verbindungen mit einer Aldehydgruppe und drei Doppelbindungen erhalten, beispielsweise eine Mischung von 4,8,11-Dodecatrienalen. Besonders bevorzugt wird eine Mischung von isomeren 4,8,11-Dodecatrienalen erhalten, enthaltend die cis,trans-, trans,cis-, trans,trans-Isomere, beispielsweise in den Mengen 50 Gew.-% cis,trans, 45 Gew.-% trans,cis und 5 Gew.-% trans,trans. Das cis,trans-Isomer ist als Verbindung (XIII) abgebildet

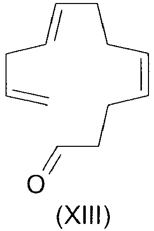

Die vorliegende Erfindung betrifft auch das erfindungsgemäße Verfahren, wobei die mindestens eine Verbindung mit Z-1 Cyclen und 7 bis 16 C-Atomen mit mindestens einer Aldehydgruppe und mindestens zwei C-C-Doppelbindungen ein 4,8,11-Dodecatrienal ist.

In einer bevorzugten Ausführungsform des erfindungsgemäßen Verfahrens enthält die in Stufe (a1) erhaltene Zusammensetzung (A1) zusätzlich mindestens eine cyclische Verbindung mit Z Cyclen und 7 bis 16 C-Atomen mit mindestens zwei Ketogruppen. Diese Verbindung entsteht aus dem als Edukt eingesetzten Olefin mit mindestens zwei Doppelbindungen durch Oxidation von zwei der vorliegenden Doppelbindungen mit Distickstoffmonoxid.

Für den besonders bevorzugten Fall, dass in das erfindungsgemäße Verfahren als Edukt 1,5,9-Cyclododecatrien, insbesondere cis,trans,trans-1,5,9-Cyclododecatrien, eingesetzt wird, wird als mindestens eine cyclische Verbindung mit Z Cyclen und 7 bis 16 C-Atomen mit mindestens zwei Ketogruppen besonders bevorzugt ein Isomerengemisch von cyclischen Verbindungen mit zwei Ketofunktionen und einer Doppelbindung erhalten, insbesondere Cyclododecendione, beispielsweise eine Mischung der 8-cis-Cyclododecen-1,5-dion-, 9-cis-Cyclododecen-1,6-dion-, 8-cis-Cyclododecen-1,4-dion-, 8-trans-Cyclododecen-1,5-dion-, 8-trans-Cyclododecen-1,4-dion- und 9-trans-Cyclododecen-1,6-dion-Isomere, beispielsweise im ungefähren Verhältnis von 38 : 19 : 19 : 12 : 6 : 6. Das als Hauptisomer gebildete 8-cis-Cyclododecen-1,5-dion ist als Verbindung (XII) abgebildet

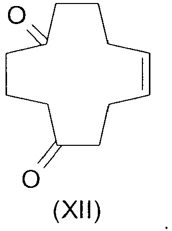

Neben dem genannten gewünschten Produkt, den genannten Nebenprodukten und nicht umgesetztem Edukt enthält die Zusammensetzung (A1) üblicherweise weitere Verbindungen, insbesondere organische Verbindungen, beispielsweise organische Verbindungen mit sauerstoffhaltigen Gruppen, beispielsweise Alkohole, Aldehyde oder Epoxide. Dabei können die organischen Verbindungen insbesondere dieselbe Anzahl oder eine abweichende Anzahl an C-Atomen aufweisen, wie der in der Zusammensetzung (A1) enthaltene cyclische Aldehyd. In der Zusammensetzung (A1) können zusätzlich zu den genannten Komponenten nicht umgesetztes Distickstoffmonoxid und gebildeter Stickstoff vorliegen.

Im Rahmen der vorliegenden Erfindung kann in Stufe (a1) Distickstoffmonoxid in reiner Form oder in Form eines Gasgemisches enthaltend Distickstoffmonoxid eingesetzt werden.

Grundsätzlich kann in Stufe (a1) des erfindungsgemäßen Verfahrens jedes Gasgemisch enthaltend Distickstoffmonoxid eingesetzt werden. Erfindungsgemäß ist es auch möglich, das Gasgemisch enthaltend Distickstoffmonoxid vor Einsatz in Stufe (a) zu reinigen oder aufzukonzentrieren. Ein geeignetes Reinigungsverfahren umfasst beispielsweise die Absorption des Gasgemisches in einem organischen Lösungsmittel oder Wasser, die Desorption des Gasgemisches aus dem beladenen organischen Lösungsmittel oder dem beladenen Wasser und das Einstellen des Gehalts an Stickoxiden NOₓ in dem Gasgemisch auf höchstens 0,01 bis 0,001 Vol.-%, bezogen auf das Gesamtvolumen des Gasgemisches. Ein derartiges Verfahren ist beispielsweise in DE 10 2004 046 167.8 beschrieben, deren diesbezüglicher Inhalt vollumfänglich in den Kontext der vorliegenden Anmeldung aufgenommen wird.

Dabei kann das eingesetzte Gasgemisch enthaltend Distickstoffmonoxid grundsätzlich aus jeder beliebigen Quelle stammen. Insbesondere ist es möglich, dass als Distickstoffmonoxidquelle das Abgas eines Verfahrens eingesetzt wird wie in WO 2006/032502, WO 2007/060160 und WO 2008/071632, und in den noch nicht veröffentlichten Anmeldungen EP 08153953.8 und EP 08153952.0 beschrieben.

Der Begriff "Gasgemisch", wie er im Rahmen der vorliegenden Erfindung verwendet wird, bezeichnet ein Gemisch aus zwei oder mehr Verbindungen, die sich bei Umgebungsdruck und Umgebungstemperatur im gasförmigen Zustand befinden. Bei veränderter Temperatur oder verändertem Druck kann das Gasgemisch auch in einem anderen Aggregatzustand vorliegen, beispielsweise flüssig, und wird im Rahmen der vorliegenden Erfindung weiter als Gasgemisch bezeichnet.
Erfindungsgemäß kann auch ein Gemisch verschiedener Abgase eingesetzt werden.

Gemäß einer weiter bevorzugten Ausführungsform der vorliegenden Erfindung stammt das mindestens eine Distickstoffmonoxid enthaltende Abgas aus einer Adipinsäureanlage, einer Dodecandisäureanlage, einer Hydroxylaminanlage und/oder einer Salpetersäureanlage, wobei letztere wiederum bevorzugt mit mindestens einem Abgas einer Adipinsäureanlage, einer Dodecandisäureanlage, einer Glyoxalanlage oder einer Hydroxylaminanlage betrieben wird.

Erfindungsgemäß kann das Gasgemisch gasförmig eingesetzt werden. Es ist jedoch auch möglich, das Gasgemisch enthaltend Distickstoffmonoxid zunächst derart zu behandeln, dass das Gasgemisch bzw. Distickstoffmonoxid in flüssiger oder überkritischer Form vorliegt und dann eingesetzt wird. Das Gasgemisch bzw. Distickstoffmonoxid kann durch geeignete Wahl des Drucks oder der Temperatur verflüssigt werden. Ebenso ist es im Rahmen der vorliegenden Erfindung möglich, das Gasgemisch in ein Lösungsmittel einzulösen.

Die Umsetzung des mindestens einen cyclischen Olefins mit Z Cyclen und 7 bis 16 C-Atomen und mindestens zwei C-C-Doppelbindungen mit Distickstoffmonoxid gemäß Schritt (a1) kann grundsätzlich in Anwesenheit eines Katalysators, jedoch auch ohne Zusatz eines Katalysators, erfolgen.

Die in Stufe (a1) erhaltene Zusammensetzung (A1) wir im Anschluss an Stufe (a1) in der erfindungsgemäßen Stufe (a2) behandelt.

In einer bevorzugten Ausführungsform wird die aus Stufe (a1) erhaltene Zusammensetzung (A1) in einer Stufe (a1b) entspannt, um noch vorhandene gasförmige Edukte oder Produkte, beispielsweise nicht umgesetztes N₂O oder gebildetes N₂, zu entfernen, bevor Zusammensetzung (A1) in Stufe (a2) eingesetzt wird. Das Entspannen kann nach dem Fachmann bekannten Verfahren erfolgen, beispielsweise durch Überführen der Zusammensetzung (A1) in einen Raum, in dem ein niedrigerer Druck herrscht.

Somit umfasst das erfindungsgemäße Verfahren bevorzugt einen Schritt (a1b):
(a1b) Entspannen der Zusammensetzung (A1), um Distickstoffmonoxid und Stickstoff zu entfernen, um eine Zusammensetzung (A1) zu erhalten, die im Wesentlichen frei von Distickstoffmonoxid und Stickstoff ist.

### Stufe (a2):

In Stufe (a2) des erfindungsgemäßen Verfahrens wird Edukt des erfindungsgemäßen Verfahrens, welches in der Oxidationsreaktion in Stufe (a1) nicht umgesetzt worden ist, von der Zusammensetzung (a1) abgetrennt, um die Zusammensetzung (A2) zu erhalten.

Stufe (a2) kann nach allen dem Fachmann bekannten Methoden erfolgen. In einer bevorzugten Ausführungsform wird in Stufe (a2) des erfindungsgemäßen Verfahrens eine Destillation durchgeführt, um beispielsweise nicht umgesetztes Edukt, d. h. mindestens ein cyclisches Olefin mit Z Cyclen und 7 bis 16 C-Atomen mit mindestens zwei C-C-Doppelbindungen, aus dem Produktstrom abzutrennen, um dieses bevorzugt in Stufe (a1) des erfindungsgemäßen Verfahrens rückzuführen.

In einer bevorzugten Ausführungsform wird für die Destillation in Stufe (a2) eine einfache Destillationskolonne mit dem Fachmann bekannten Packungen verwendet. Die Destillation in Stufe (a2) des erfindungsgemäßen Verfahrens wird bevorzugt im Vakuum, beispielsweise bei einem Druck ≤ 1000 mbar, bevorzugt ≤ 500 mbar, besonders bevorzugt ≤ 300 mbar, durchgeführt. Für den erfindungsgemäß bevorzugten Fall, dass als Edukt eine olefinische Verbindung mit 12 C-Atomen eingesetzt wird, wird Stufe (a2) bevorzugt bei einem Druck ≤ 120 mbar, besonders bevorzugt ≤ 70 mbar, ganz besonders bevorzugt ≤ 60 mbar, durchgeführt. Erfindungsgemäß können dem Fachmann bekannte Destillationskolonnen eingesetzt werden, bevorzugt sind solche, die mindestens 20, bevorzugt mindestens 25, besonders bevorzugt mindestens 30 theoretische Trennstufen aufweisen. In einer weiteren bevorzugten Ausführungsform befinden sich 35 bis 55% der Trennstufen im Abtriebsteil der Destillationskolonne. Das Rücklaufverhältnis beträgt in der bevorzugten Ausführungsform, dass eine olefinische Verbindung mit 12 C-Atomen als Edukt eingesetzt wird, 1 bis 2, bevorzugt 1,2 bis 1,8. Für die anderen genannten Edukte kann das Rücklaufverhältnis durch den Fachmann angepasst werden.

Als Kopfprodukt dieser Destillation wird im Wesentlichen reines Edukt, d. h. mindestens ein cyclisches Olefin mit Z Cyclen und 7 bis 16 C-Atomen mit mindestens zwei C-C-Doppelbindungen, erhalten, welches in einer besonders bevorzugten Ausführungsform als Substrat in Stufe (a1) des erfindungsgemäßen Verfahrens zurückgeführt wird. In einer bevorzugten Ausführungsform betrifft die vorliegende Erfindung das erfindungsgemäße Verfahren, wobei das in Stufe (a2) abgetrennte mindestens eine cyclische Olefin mit Z Cyclen und 7 bis 16 C-Atomen mit mindestens zwei C-C-Doppelbindungen wieder in Stufe (a1) zurückgeführt wird.

Das in der beschriebenen Destillation in Stufe (a2) erhaltene Sumpfprodukt entspricht im Wesentlichen der oben beschriebenen Zusammensetzung (A2).

Das erfindungsgemäß gewünschte Produkt, die mindestens eine Verbindung mit Z-1 Cyclen und 7 bis 16 C-Atomen mit mindestens einer Aldehydgruppe und mindestens zwei C-C-Doppelbindungen, liegt in der Zusammensetzung (A2) im Allgemeinen in einer Menge von 0,1 bis 50,0 Gew.-%, bevorzugt 0,5 bis 10,0 Gew.-%, besonders bevorzugt 1,0 bis 5,0 Gew.-%, vor.

In der bevorzugten Ausführungsform des erfindungsgemäßen Verfahrens, dass die Zusammensetzung (A1) auch mindestens eine cyclische Verbindung mit Z Cyclen und 7 bis 16 C-Atomen und mindestens zwei Ketogruppen enthält, wird in Stufe (a2) des erfindungsgemäßen Verfahrens eine Zusammensetzung (A2) erhalten, mindestens enthaltend
- die cyclische Verbindung mit Z Cyclen und 7 bis 16 C-Atomen mit einer Ketogruppe und
- die Verbindung mit Z-1 Cyclen und 7 bis 16 C-Atomen mit mindestens einer Aldehydgruppe und mindestens zwei C-C-Doppelbindungen und
- mindestens eine cyclische Verbindung mit Z Cyclen und 7 bis 16 C-Atomen mit mindestens zwei Ketogruppen.

Die mindestens eine cyclische Verbindung mit Z Cyclen und 7 bis 16 C-Atomen mit mindestens zwei Ketogruppen liegt in einer bevorzugten Ausführungsform in der Zusammensetzung (A2) im Allgemeinen in einer Menge von 0,1 bis 20,0 Gew.-%, bevorzugt 0,5 bis 10,0 Gew.-%, besonders bevorzugt 1,0 bis 5,0 Gew.-%, vor.

In einer weiteren Ausführungsform des erfindungsgemäßen Verfahrens kann Stufe (a2) auch so ausgeführt werden, dass das gewünschte Produkt, die Verbindung mit Z-1 Cyclen und 7 bis 16 C-Atomen mit mindestens einer Aldehydgruppe in der oben beschriebenen Destillationskolonne als Seitenabzug erhalten wird. Dabei bleibt das in diesem Schritt abzutrennende Edukt, d. h. das Olefin mit Z Cyclen und 7 bis 16 C-Atomen und mindestens zwei C-C-Doppelbindungen Kopfprodukt von Stufe (a1). Die als Seitenabzug erhaltene Verbindung mit Z-1 Cyclen und 7 bis 16 C-Atomen mit mindestens einer Aldehydgruppe und mindestens zwei C-C-Doppelbindungen enthält in diesem Fall zusätzlich in Stufe (a1) nicht umgesetztes Edukt, d. h. ein cyclisches Olefin mit Z Cyclen und 7 bis 16 C-Atomen und mindestens zwei C-C-Doppelbindungen, beispielsweise in einer Menge von 0,1 bis 5 Gew.-%, bevorzugt 1 bis 3 Gew.-%. Die als Seitenabzug erhaltene Fraktion kann in einer bevorzugten Ausführungsform direkt in Stufe (b2) eingesetzt werden, um das gewünschte Produkt in einer für Riechstoffe notwendigen Reinheit zu gewinnen.

### Stufe (b1):

Das Abtrennen der cyclischen Verbindung mit Z Cyclen und 7 bis 16 C-Atomen mit einer Ketogruppe in Schritt (b1) des erfindungsgemäßen Verfahrens erfolgt destillativ.

Dabei wird Stufe (b1) des erfindungsgemäßen Verfahrens in mindestens zwei Kolonnen durchgeführt. Dabei wird Zusammensetzung (A2) aus Schritt (a2) in einem ersten Schritt in einer einfachen Destillationskolonne (T1) behandelt. Dabei erhält man bevorzugt einen Kopfstrom (K1), der im Wesentlichen aus mindestens einer Verbindung mit Z-1 Cyclen und 7 bis 16 C-Atomen mit mindestens einer Aldehydgruppe und mindestens zwei C-C-Doppelbindungen besteht, und gegebenenfalls maximal 35 Gew.-%, bevorzugt maximal 30 Gew.-%, besonders bevorzugt maximal 25 Gew.-% mindestens eine cyclische Verbindung mit Z Cyclen und 7 bis 16 C-Atomen mit einer Ketogruppe enthält. Des Weiteren wird ein Sumpfstrom (S1) erhalten, der alle restlichen Komponenten enthalten kann, unter anderem auch die mindestens eine Verbindung mit Z Cyclen und 7 bis 16 C-Atomen und einer Ketogruppe und die mindestens eine cyclische Verbindung mit Z Cyclen und 7 bis 16 C-Atomen mit mindestens zwei Ketogruppen.

Als Destillationskolonne (T1) können alle dem Fachmann als geeignet bekannten Kolonnen verwendet werden. Dabei enthält die Kolonne (T1) mindestens 15 theoretische Trennstufen, besonders bevorzugt mindestens 20. Dabei ist es weiterhin bevorzugt, dass die meisten Trennstufen, beispielsweise mindestens 50% der Trennstufen, im Verstärkungsteil der Kolonne vorliegen. Die Destillation in der Destillationskolonne (T1) wird bevorzugt bei einem Druck unterhalb von Atmosphärendruck durchgeführt, beispielsweise, insbesondere für den bevorzugten Fall, dass als Edukt eine olefinische Verbindung mit 12 C-Atomen eingesetzt wird, bei einem Kopfdruck von weniger als 50 mbar, besonders bevorzugt weniger als 20 mbar. Für die anderen erfindungsgemäß einsetzbaren Verbindungen ist ein geeigneter Druck durch den Fachmann zu ermitteln. Die Destillation in der Destillationskolonne (T1) wird, für den bevorzugten Fall, dass als Edukt eine olefinische Verbindung mit 12 C-Atomen eingesetzt wird, bevorzugt bei einer Sumpftemperatur von 120 bis 220 °C, besonders bevorzugt 150 bis 200 °C, durchgeführt. Für die anderen erfindungsgemäß geeigneten Edukte kann die Destillationstemperatur, auch in Abhängigkeit vom eingestellten Druck, durch den Fachmann gewählt werden.

Bevorzugt wird der Sumpfstrom (S1) aus der ersten Destillationskolonne (T1) in mindestens einer weiteren einfachen Destillationskolonne (T2) behandelt. In einer bevorzugten Ausführungsform wird dabei ein Kopfstrom (K2) erhalten, der im Wesentlichen mindestens eine cyclische Verbindung mit Z Cyclen und 7 bis 16 C-Atomen mit mindestens einer Ketogruppen enthält. Dieser Kopfstrom (K2) enthält in einer bevorzugten Ausführungsform im Wesentlichen keine Verbindung mit Z-1 Cyclen und 7 bis 16 C-Atomen mit mindestens einer Aldehydgruppe und mindestens zwei C-C-Doppel-bindungen, d.h. weniger als 1,0 Gew.-%, in einer bevorzugten Ausführungsform höchstens 0,2 Gew.-%. In der zweiten Destillation in T2 des erfindungsgemäßen Verfahrens (Schritt (b1)) wird des Weiteren ein Sumpfstrom (S2) erhalten, der mindestens eine cyclische Verbindung mit Z Cyclen und 7 bis 16 C-Atomen mit mindestens zwei Ketogruppen und andere Nebenprodukte der Oxidation gemäß Schritt (a1) enthält, jedoch maximal 40 Gew.-%, bevorzugt maximal 25 Gew.-% der mindestens einen cyclischen Verbindung mit Z Cyclen und 7 bis 16 C-Atomen mit einer Ketogruppe enthält.

Als Destillationskolonne (T2) kann jede Kolonne eingesetzt werden, die dem Fachmann als geeignet bekannt ist. Dabei weist die Kolonne mindestens 30 Trennstufen auf, besonders bevorzugt mindestens 35 Trennstufen. In einer weiteren bevorzugten Ausführungsform liegen die meisten Trennstufen im Abtriebsteil vor, besonders bevorzugt liegen mindestens 28 theoretische Trennstufen im Abtriebsteil vor. Die Destillation in Trennkolonne (T2) erfolgt bevorzugt bei einem Druck unterhalb von Atmosphärendruck, für den bevorzugten Fall, dass eine olefinische Verbindung mit 12 C-Atomen als Edukt eingesetzt wird, beispielsweise bei einem Kopfdruck ≤ 50 mbar, besonders bevorzugt ≤ 25 mbar. Die Destillation in der Destillationskolonne (T2) wird für den bevorzugten Fall, dass eine olefinische Verbindung mit 12 C-Atomen eingesetzt wird, bevorzugt bei einer Sumpftemperatur von 120 bis 220 °C, besonders bevorzugt 150 bis 200 °C, durchgeführt. Für die anderen erfindungsgemäß geeigneten Edukte kann die Temperatur, auch in Abhängigkeit vom eingestellten Druck, durch den Fachmann leicht eingestellt werden.

Erfindungsgemäß ist es auch möglich, die Destillationskolonnen (T1) und (T2) in umgekehrter Reihenfolge zu betreiben, das heißt in der ersten Kolonne die mindestens eine cyclische Verbindung mit Z Cyclen und 7 bis 16 C-Atomen mit mindestens einer Ketogruppe und die mindestens eine Verbindung mit Z-1 Cyclen und 7 bis 16 C-Atomen mit mindestens einer Aldehydgruppe und mindestens zwei C-C-Doppel-bindungen über Kopf zu trennen und in der zweiten Kolonne die mindestens eine cyclische Verbindung mit Z-1 Cyclen und 7 bis 16 C-Atomen mit mindestens einer Aldehydgruppe und mindestens zwei C-C-Doppelbindungen über Kopf abzutrennen.

Die bevorzugt eingesetzten Kolonnen (T1) und (T2) sind beispielsweise in Olujic et al., Chem. Biochem. Eng. Q. 2003, 17, Seiten 301 bis 309 beschrieben. In einer weiteren bevorzugten Ausführungsform von Schritt (b1) des erfindungsgemäßen Verfahrens sind die Kolonnen (T1) und (T2) thermisch miteinander gekoppelt, beispielsweise indem man eine Seitenkolonne ohne eigenen Verdampfer und Kondensator verwendet (Petlyuk-Schaltung).

Ganz besonders bevorzugt wird anstelle der zwei Destillationskolonnen (T1) und (T2) in Schritt (b1) des erfindungsgemäßen Verfahrens eine einzige Trennwandkolonne eingesetzt.

Erfindungsgemäß kann jede Trennwandkolonne in Schritt (b1) des erfindungsgemäßen Verfahrens eingesetzt werden, die für den Fachmann für das vorliegende Trennproblem als geeignet erscheint. Geeignete Trennwandkolonnen sind in Olujic et al., Chem. Biochem. Eng. Q. 2003, 17, Seiten 301 bis 309 genannt.

In einer bevorzugten Ausführungsform wird eine kontinuierliche Trennwandkolonne eingesetzt, die bevorzugt über mindestens drei Bereiche verfügt. Bevorzugt verfügt die Trennwandkolonne über einen unteren Bereich, der bevorzugt mindestens 2, besonders bevorzugt mindestens 4 theoretische Böden aufweist. Des Weiteren weist die bevorzugt eingesetzte Trennwandkolonne einen mittleren Teil auf, der bevorzugt mindestens 15, besonders bevorzugt mindestens 25, theoretische Böden aufweist. In einer weiteren bevorzugten Ausführungsform weist die eingesetzte Trennwandkolonne einen oberen Teil auf, der bevorzugt mindestens 4, besonders bevorzugt mindestens 7, theoretische Böden aufweist. Der mittlere Teil ist durch eine bevorzugt mittig angeordnete Trennwand in einen Eingangs- und einen Ausgangsbereich unterteilt.

In einer weiteren bevorzugten Ausführungsform ist die Trennwandkolonne mit einer geeigneten Packung bestückt. Geeignete Kolonnenpackungen sind dem Fachmann bekannt, beispielsweise aus J. F. Fair in "Handbook of Separation Process Technology", R. W. Rousseau (Hrsg.), (1987), John Wiley & Sons, Seiten 295 - 312.

Um die Temperatur bei der Destillation in der Trennwandkolonne möglichst niedrig halten zu können, wird bevorzugt bei einem Druck unterhalb von Atmosphärendruck gearbeitet, beispielsweise bei einem Druck innerhalb der Trennwandkolonne von weniger als 500 mbar, bevorzugt weniger als 200 mbar, insbesondere weniger als 100 mbar und ganz besonders bevorzugt weniger als 50 mbar. Der Druckunterschied zwischen Kolonnensumpf und Kolonnenkopf beträgt bevorzugt weniger als 50 mbar. In einer bevorzugten Ausführungsform beträgt der Kopfdruck in der Trennwandkolonne 0,1 bis 100 mbar, besonders bevorzugt 3 bis 50 bar.

In der Trennwandkolonne wird die Destillation bevorzugt bei einer Sumpftemperatur von 150 bis 220 °C, besonders bevorzugt 160 bis 200 °C durchgeführt. Diese Werte gelten insbesondere für den bevorzugten Fall, dass eine olefinische Verbindung mit 12 C-Atomen als Edukt eingesetzt wird, für die anderen erfindungsgemäß einsetzbaren Edukte können die Werte durch den Fachmann entsprechend angepasst werden.

Über Kopf der Trennwandkolonne werden bevorzugt die leichtsiedenden Komponenten der Zusammensetzung (B1) als Kopfstrom (K3) abgetrennt. Im Allgemeinen enthält der Kopfstrom (K3) das gewünschte Produkt des erfindungsgemäßen Verfahrens, d. h. mindestens eine Verbindung mit Z-1 Cyclen und 7 bis 16 C-Atomen mit mindestens einer Aldehydgruppe und mindestens zwei C-C-Doppelbindungen. Der Kopfstrom (K3) enthält im Allgemeinen mindestens 50 Gew.-%, besonders bevorzugt mindestens 70 Gew.-%, ganz besonders bevorzugt mindestens 75 Gew.-%, mindestens eine Verbindung mit Z-1 Cyclen und 7 bis 16 C-Atomen und mindestens einer Aldehydgruppe und mindestens zwei C-C-Doppelbindungen. Kopfstrom (K3) kann zusätzlich zu dem gewünschten Produkt auch mindestens ein cyclisches Olefin mit Z Cyclen und 7 bis 16 C-Atomen und mindestens zwei C-C-Bindungen, d. h. in Schritt (a1) nicht umgesetztes Edukt, und gegebenenfalls maximal 35 Gew.-%, bevorzugt maximal 30 Gew.-%, besonders bevorzugt maximal 25 Gew.-% mindestens eine cyclische Verbindung mit Z Cyclen und 7 bis 16 C-Atomen mit einer Ketogruppe enthalten.

Als Sumpf der Trennwandkolonne werden bevorzugt die hochsiedenden Komponenten der Zusammensetzung (A2) als Sumpfstrom (S2) abgetrennt. In einer bevorzugten Ausführungsform enthält Sumpfstrom (S2) mindestens eine cyclische Verbindung mit Z Cyclen und 7 bis 16 C-Atomen mit mindestens zwei Ketogruppen und andere Nebenprodukte der Oxidation gemäß Schritt (a1). (S2) enthält im Allgemeinen maximal 40 Gew.-%, bevorzugt maximal 25 Gew.-% der mindestens einen cyclischen Verbindung mit Z Cyclen und 7 bis 16 C-Atomen mit einer Ketogruppe.

Über Seitenabzug der Trennwandkolonne wird eine Zusammensetzung erhalten, die im Wesentlichen mindestens eine cyclische Verbindung mit Z Cyclen und 7 bis 16 C-Atomen mit einer Ketogruppe enthält. Des Weiteren können in dieser Zusammensetzung gegebenenfalls mindestens eine cyclische Verbindung mit Z Cyclen und 7 bis 16 C-Atomen mit mindestens zwei Ketogruppen und mindestens eine Verbindung mit Z-1 Cyclen 7 bis 16 C-Atomen mit mindestens einer Aldehydgruppe und mindestens zwei C-C-Doppelbindungen vorliegen. Bevorzugt wird die Destillation so durchgeführt, dass die Menge dieser zwei Nebenkomponenten im Seitenabzug minimiert wird. Konkret wird die Destillation so durchgeführt, dass im Seitenabzug weniger als 0,5 Gew.-% der mindestens eine cyclische Verbindung mit Z Cyclen und 7 bis 16 C-Atomen mit mindestens zwei Ketogruppen und weniger als 1 Gew.-% der mindestens eine Verbindung mit Z-1 Cyclen 7 bis 16 C-Atomen mit mindestens einer Aldehydgruppe und mindestens zwei C-C-Doppelbindungen enthalten sind.

In dem Kopfstrom (K3), welcher im Wesentlichen das gewünschte Produkt, mindestens eine Verbindung mit Z-1 Cyclen mit 7 bis 16 C-Atomen mit mindestens einer Aldehydgruppe und mindestens zwei C-C-Doppelbindungen, enthält, kann zusätzlich in Stufe (a1) nicht umgesetztes Edukt, d. h. mindestens ein cyclisches Olefin mit Z Cyclen und 7 bis 16 C-Atomen und mindestens zwei C-C-Doppelbindungen, vorliegen, beispielsweise in einer Menge von 0,1 bis 20 Gew.-%, bevorzugt 1 bis 15 Gew.-%. Für die Verwendung des erfindungsgemäßen Produktes als Riechstoff ist im Allgemeinen ein Gehalt an mindestens einem cyclischen Olefin mit Z Cyclen und 7 bis 16 C-Atomen mit mindestens zwei C-C-Doppelbindungen von 0 bis 5 Gew.-%, bevorzugt 0 bis 1 Gew.-%, besonders bevorzugt 0 bis 0,1 Gew.-%, ganz besonders bevorzugt 0 bis 0,05 Gew.-%, notwendig. Daher wird das erfindungsgemäß als Kopfstrom (K3) erhaltene Produkt in einer bevorzugten Ausführungsform in einem folgenden Schritt aufgereinigt.

Die vorliegende Erfindung betrifft in einer bevorzugten Ausführungsform auch das erfindungsgemäße Verfahren, wobei die in Schritt (b1) abgetrennte mindestens eine cyclische Verbindung mit Z Cyclen und 7 bis 16 C-Atomen mit einer Ketogruppe mindestens eine C-C-Doppelbindung aufweist.

In einer weiteren bevorzugten Ausführungsform betrifft die vorliegende Erfindung auch das erfindungsgemäße Verfahren, wobei die mindestens eine cyclische Verbindung mit Z Cyclen und 7 bis 16 C-Atomen mit einer Ketogruppe mit mindestens einer C-C-Doppelbindung nach Schritt (b1) zu mindestens einer gesättigten cyclischen Verbindung mit Z Cyclen und 7 bis 16 C-Atomen mit einer Ketogruppe hydriert wird. Entsprechende Verfahren zur Hydrierung sind dem Fachmann bekannt.

Die Reinigung in dem Schritt (b2) des erfindungsgemäßen Verfahrens kann nach allen dem Fachmann bekannten Verfahren erfolgen. In einer bevorzugten Ausführungsform erfolgt die Reinigung in Schritt (B2) durch eine Destillation. Diese Destillation kann kontinuierlich oder diskontinuierlich, d. h. im Batchverfahren, durchgeführt werden. Bevorzugt wird die Destillation in Schritt (b2) im Batchverfahren durchgeführt.

In einer besonders bevorzugten Ausführungsform wird Stufe (b2) des erfindungsgemäßen Verfahrens in einer Destillationsblase durchgeführt, die über eine aufgesetzte Kolonne verfügt. Besonders bevorzugt weist die Kolonne mindestens 20 Trennstufen auf. In einer weiteren bevorzugten Ausführungsform wird die Destillation in Stufe (b2) des erfindungsgemäßen Verfahrens bei vermindertem Druck, d. h. bei einem Druck unterhalb Atmosphärendruck, durchgeführt. Für den bevorzugten Fall, dass in dem erfindungsgemäßen Verfahren ein Olefin mit 12 C-Atomen eingesetzt wird, wird diese Destillation bei einem Kopfdruck von 0,1 bis 100 mbar, ganz besonders bevorzugt 3 bis 50 mbar, durchgeführt. Für die anderen erfindungsgemäß einsetzbaren Edukte muss der Destillationsdruck durch den Fachmann entsprechend angepasst werden. Bei der Durchführung der Destillation im Batchverfahren werden im Allgemeinen zunächst Fraktionen erhalten, die noch mindestens ein cyclisches Olefin mit Z Cyclen mit 7 bis 16 C-Atomen und mindestens zwei C-C-Doppelbindungen enthalten. Daran anschließend werden Fraktionen erhalten, die das gewünschte Produkt in der entsprechenden Reinheit enthalten.

Nach Stufe (b2) des erfindungsgemäßen Verfahrens wird eine Mischung erhalten, die im Allgemeinen 92 bis 99,8 Gew.-%, bevorzugt 94 bis 99,5 Gew.-%, besonders bevorzugt 95 bis 99,2 Gew.-%, ganz besonders bevorzugt 96 bis 99 Gew.-%, der mindestens einen Verbindung mit Z-1 Cyclen und 7 bis 16 C-Atomen mit mindestens einer Aldehydgruppe und mindestens zwei C-C-Doppelbindungen, bevorzugt mindestens ein 4,8,11-Dodecatrienal, und 0,0001 bis 5 Gew.-%, bevorzugt 0,001 bis 2 Gew.-%, besonders bevorzugt 0,01 bis 0,5 Gew.-%, ganz besonders bevorzugt 0,05 bis 0,4 Gew.-%, des mindestens einen cyclischen Olefins mit Z Cyclen und 7 bis 16 C-Atomen und mindestens zwei C-C-Doppelbindungen enthält, bevorzugt mindestens ein 1,5,9-Cyclododecatrien. Als Rest zu 100 Gew.-% liegen in der Mischung noch weitere nicht identifizierbare Verbindungen in kleinen Mengen, beispielsweise bis zu 2 Gew.-%, vor. Die Summe der Mengen der Verbindung mit Z-1 Cyclen und 7 bis 16 C-Atomen mit mindestens einer Aldehydgruppe und mindestens zwei C-C-Doppelbindungen, des mindestens einen cyclischen Olefins mit Z Cyclen und 7 bis 16 C-Atomen und mindestens zwei C-C-Doppelbindungen und der weiteren nicht identifizierbaren Verbindungen, d. h. aus dem erfindungsgemäßen Verfahren stammenden Nebenprodukten, ergibt jeweils 100 Gew.-%. Die Summe der Mengen der einzelnen Komponenten übersteigt 100 Gew.-% nicht. Die Zusammensetzungen können durch dem Fachmann bekannte Verfahren bestimmt werden, beispielsweise Gaschromatographie.

Die erfindungsgemäß hergestellte Mischung eignet sich als Riechstoff. Die erfindungsgemäß hergestellte Mischung weist den Duft von Tannennadeln und Holz auf.

Daher betrifft die vorliegende Erfindung auch die Verwendung einer Mischung, enthaltend 92 bis 99,8 Gew.-% mindestens eines 4,8,11-Dodecatrienals und 0,0001 bis 5 Gew.-% 1,5,9-Cyclododecatriene, als Riechstoff, beispielsweise in Parfüms, Kosmetika, Seifen, Reinigungsmitteln, Shampoos, Nahrungsmitteln, Hygieneprodukten oder Pharmazeutika.

Die Mischung liegt bei den erfindungsgemäßen Anwendungen in üblichen Mengen vor, beispielsweise 0,0001 bis 5 Gew.-%. Es ist möglich, dass die Mischung allein als Riechstoff vorliegt. Es ist darüber hinaus auch möglich, dass die Mischung in Mischung mit weiteren Riechstoffen und/oder weiteren dem Fachmann bekannten Additiven, beispielsweise Stabilisatoren, Emulgatoren, Tenside oder Farbstoffen verwendet werden.

Die in der erfindungsgemäßen Stufe (b1) abgetrennte mindestens eine cyclische Verbindung mit Z Cyclen und 7 bis 16 C-Atomen mit einer Ketogruppe kann gegebenenfalls nach dem Fachmann bekannten Verfahren weiter behandelt werden, beispielsweise Hydrierung, Behandlung mit Basen, Brønstedt-Säuren und/oder Lewis-Säuren, destillative Behandlung. Geeignete Verfahren sind beispielsweise in WO 2008/000757 A1, WO 2008/000756 A1, WO 2005/030690 A2 und WO 2008/000754 A1 beschrieben.

Im Folgenden wird die vorliegende Erfindung anhand von Beispielen näher erläutert.

### Beispiele:

### Beispiel 1: Oxidation von 1,5,9-Cyclododecatrien (CDT) mit N₂O

Aus entsprechenden Vorlagebehältern und mittels geeigneten Dosierpumpen werden 2000 g/h 1,5,9-Cyclododecatrien und 68 g/h flüssiges N₂O über einen statischen Mischer in einen Rohrreaktor (Doppelmantelrohr, gewickelt, Ø innen= 6 mm, Länge 36 m) gepumpt. Das Rohr wird mittels Wärmeträgeröl, dass im Doppelmantel im Gleichstrom zum Produkt fließt auf 280 °C thermostatisiert, wobei die Ölausgangstemperatur weniger als 2 °C über die Öleingangstemperatur liegt. Der Reaktionsdruck wird mittels einen Druckregelventil am Reaktorausgang auf 100 bar geregelt. Der Umsatz an 1,5,9-Cyclododecatrien am Reaktorausgang beträgt 11,3%. Nach Passieren der Reaktionszone wird das Reaktionsgemisch in zwei unisolierten Flashbehältern zunächst auf 3 bar und anschließend auf 60 mbar entspannt, um den gebildeten N₂ und nicht umgesetztes N₂O abzuführen. Dabei kühlt sich das Produkt auf unter 100 °C ab. Das flüssige Produkt wird dann in einer Packungskolonne mit mindestens 7 theoretischen Trennstufen bei 60 mbar destilliert (T(Sumpf) = 170 °C, T(Kopf) = 130 °C). Als Kopfprodukt erhält man nicht umgesetztes 1,5,9-Cyclododecatrien mit einer Reinheit >99%, das wieder in die Reaktion zurückgeführt wird. Der Sumpfaustrag ist eine nur leicht gelbliche Flüssigkeit und hat die in Tabelle 1 aufgeführte Zusammensetzung, bestimmt per Gaschromatographie.

**Tabelle 1**

| **Verbindung** | **Menge [Gew.-%]** |
|---|---|
| Cyclododeca-4,8-dienone | 92,0 |
| 4,8,11-Dodecatrienale | 2,3 |
| Cyclododecendione | 2,2 |
| Cyclododecendione | 2,2 |
| Cycloundeca-3,7-diencarbaldehyd | 1,0 |
| 1,5,9-Cyclododecatrien | 0,4 |
| trans-1,2-Epoxy-cis,trans-5,9-cyclododecadien | 0,01 |
| Dimere | 2,0 |
| andere, nicht identifiziert | Rest zu 100 |

Das Produkt wird gesammelt und in Beispiel 2 eingesetzt.

### Beispiel 2: Isolierung von 4,8,11-Dodecatrienal als Rohware

Für die Destillation des Produktgemisches aus Beispiel 1 wird eine kontinuierliche Labortrennwandkolonne mit 64 mm Innendurchmesser und einer Länge von 2,6 m (damit ist die Gesamtlänge der Packung gemeint). In Vorversuchen mit Testgemischen wird ermittelt, dass die Kolonne 35 theoretische Trennstufen hat. Die Kolonne ist in drei Bereiche unterteilt. Der untere Bereich (Stufen 1 bis 9) hat eine Länge von 0,65 m. Der mittlere Bereich (Stufen 9 bis 27) ist 1,3 m lang und durch eine mittig angeordnete Trennwand in eine Eingangs- und einer Ausgangsseite geteilt. Auf der Eingangsseite ist der Zulauf auf der Höhe von Stufe 19 angebracht. Auf der Ausgangsseite wird auf der Höhe der Stufe 12 das Seitenstromprodukt gasförmig abgenommen. Der obere Bereich (Stufen 27 bis 35) hat eine Länge von 0,65 m. Die gesamte Kolonne ist mit einer Packung bestückt (Montz A3 750). Die Destillation wird bei einem Kopfdruck von ca. 44 mbar durchgeführt, der Druckverlust über die Packung beträgt 3,6 mbar. Um die Verweilzeit und damit die thermische Belastung im Sumpf zu minimieren, wird als Sumpfverdampfer ein Sambay-Verdampfer ("wiped film evaporator") eingesetzt. Die Kopftemperatur beträgt 137 °C und die Sumpftemperatur 185 °C. Mit einer Dosierpumpe werden 501 g/h des zu destillierenden Gemisches dosiert, wobei das Gemisch vorher auf 180 °C erhitzt wird. Über den Seitenstrom werden 481 g/h Produkt erhalten, das die in Tabelle aufgeführte Zusammensetzung aufweist, bestimmt per Gaschromatographie.

**Tabelle 2**

| **Verbindung** | **Menge [Gew.-%]** |
|---|---|
| Cyclododeca-4,8-dienone | 98,5 |
| 4,8,11-Dodecatrienale | 0,1 |
| Cyclododecendione | 0,2 |
| Cycloundeca-3,7-diencarbaldehyd | 1,1 |
| trans-1,2-Epoxy-cis,trans-5,9-cyclododecadien | 0,01 |
| andere, nicht identifiziert | Rest zu 100 |

Es handelt sich hierbei um eine farblose Flüssigkeit mit einem Schmelzpunkt von +1 °C.

Im Sumpf der Destillation werden 6 g/h Sumpfprodukt als dunkelgelbe bis braune Flüssigkeit mit der in Tabelle 3 aufgeführten Zusammensetzung erhalten, bestimmt über Gaschromatographie.

**Tabelle 3**

| **Verbindung** | **Menge [Gew.-%]** |
|---|---|
| Cyclododeca-4,8-dienone | 6,7 |
| Cyclododecendione | 47,0 |
| Hochsieder | 46,0 |
| andere, nicht identifiziert | Rest zu 100 |

Im Kopf werden 14 g/h Kopfprodukt als farblose Flüssigkeit mit der in Tabelle 4 aufgeführten Zusammensetzung erhalten, bestimmt über Gaschromatographie.

**Tabelle 4**

| **Verbindung** | **Menge [Gew.-%]** |
|---|---|
| 4,8,11-Dodecatrienale | 76,0 |
| Cyclododeca-4.8-dienone | 9,9 |
| 1,5,9-Cyclododecatrien | 13,6 |
| andere, nicht identifiziert | Rest zu 100 |

Die angegebenen Flüsse sind Mittelwerte aus einer kontinuierlichen Destillation bei denen insgesamt 80 kg Zulauf verarbeitet werden.

Das Seitenstromprodukt kann dann z.B. gemäß der WO 2005/030690 bzw. der WO 2008/000754 weiter zu Cyclododecanon verarbeitet werden.

Das Kopfprodukt ist schon stark angereichert an 4,8,11-Dodecatrienalen. Das hier enthaltene 4,8,11-Dodecatrienal besteht weiterhin aus einem Gemisch von drei Isomeren, die noch im gleichen Verhältnis wie in dem Produktgemisch aus Beispiel 1 vorliegen.

### Beispiel 3: Reindestillation von 4,8,11-Dodecatrienal

770 g des Kopfproduktes aus Beispiel 2 (mit der dort angegeben Zusammensetzung) werden in einer automatisierten Batch-Destillationsanlage bei verminderten Druck rektifiziert. Die Anlage beinhaltet eine Kolonne mit 30 mm Durchmesser und 3,5 m Höhe, die mit einer Packung (Sulzer DX, Gesamtlänge: 3,17 m) gefüllt ist. Die Destillation wird bei einem konstanten Kopfdruck von 40 mbar durchgeführt und die Druckdifferenz zwischen Kopf und Sumpf der Destillationskolonne beträgt ca. 5 mbar. Das Rücklaufverhältnis wird am Anfang (bis die ersten 100 g überdestilliert sind) auf 100 eingestellt. Ab diesem Zeitpunkt wird das Rücklaufverhältnis auf 150 erhöht. Es werden immer etwa 25 g destilliert und dann, bevor eine neue Fraktion genommen wird (am Anfang oder wenn sich die Bedingungen rasch änderten werden durchaus auch kleinere Fraktionen gesammelt). Die vier Fraktionen zwischen 217 und 317 g Destillat haben alle fast die gleich Zusammensetzung und destillieren alle zwischen 138,8 und 144,0 °C Kopftemperatur. Die vier Fraktionen (farblose Flüssigkeit) enthalten 98,2 Gew.-% der gewünschten 4,8,11-Dodecatrienale als ca. 1:1-Gemisch der cis,trans- und trans,cis-Isomere (das trans,trans-Isomer hat einen höheren Siedepunkt und verbleibt im Sumpf). Als Verunreinigungen sind noch 0,26 Gew.-% 1,5,9-Cyclododecatrien und 0,06 Gew.-% 4,8-Cyclododecadienon, neben weiteren nicht identifizierten Nebenkomponenten, enthalten.

### Beispiel 4: Geruchsprobe

In ein frisch gespültes Schraubdeckelglas mit einem Volumen von 370 ml (Durchmesser: 70 mm) wird 1 ml des Produktes aus Beispiel 3 eingefüllt und das Glas wird mit einem fabrikneuen Schraubdeckel aus Kunststoff verschlossen. Das verschlossene Glas wird dann 15 Minuten bei Raumtemperatur stehen gelassen. Zum Abriechen wird das Glas nach der Equilibrierung kurzzeitig geöffnet und von dem Parfümeur geruchlich beurteilt. Der Geruchseindruck wird als intensiv nach Tannennadeln, holzig und aldehydisch riechend beschrieben.

## Patentansprüche

1. Verfahren zur Herstellung einer Verbindung mit Z-1 Cyclen und 7 bis 16 C-Atomen mit mindestens einer Aldehydgruppe, umfassend die Stufen:
(a1) Oxidation eines cyclischen Olefins mit Z Cyclen und 7 bis 16 C-Atomen und mindestens zwei C-C-Doppelbindungen, ausgewählt aus der Gruppe bestehend aus 1,5-Cyclooctadien, 1,5-Cyclododecadien, 1,9-Cyclohexadecadien, 1,8-Cyclotetradecadien, 1,6-Cyclodecadien, 1,6,11-Cyclopentadecatrien, 1,5,9-Cyclododecatrien, Vinylcyclohexen, Norbornadien, Ethylidennorbornen, mittels Distickstoffmonoxid unter Erhalt einer Zusammensetzung (A1), mindestens enthaltend
- eine cyclische Verbindung mit Z Cyclen und 7 bis 16 C-Atomen mit einer Ketogruppe,
- ein cyclisches Olefin mit Z Cyclen und 7 bis 16 C-Atomen mit mindestens zwei C-C-Doppelbindungen und
- eine Verbindung mit Z-1 Cyclen und 7 bis 16 C-Atomen mit mindestens einer Aldehydgruppe und mindestens zwei C-C-Doppelbindungen,
(a2) Abtrennen des cyclischen Olefins mit Z Cyclen und 7 bis 16 C-Atomen mit mindestens zwei C-C-Doppelbindungen aus der Zusammensetzung (A1) aus Stufe (a1), um eine Zusammensetzung (A2) zu erhalten, mindestens enthaltend
- die cyclische Verbindung mit Z Cyclen und 7 bis 16 C-Atomen mit einer Ketogruppe und
- die Verbindung mit Z-1 Cyclen und 7 bis 16 C-Atomen mit mindestens einer Aldehydgruppe und mindestens zwei C-C-Doppelbindungen,
(b1) Abtrennen der cyclischen Verbindung mit Z Cyclen und 7 bis 16 C-Atomen mit einer Ketogruppe aus der Zusammensetzung (A2) aus Schritt (a2), um eine Zusammensetzung (B1) zu erhalten, enthaltend mindestens 50 Gew.-% der Verbindung mit Z-1 Cyclen und 7 bis 16 C-Atomen mit mindestens einer Aldehydgruppe und mindestens zwei C-C-Doppelbindungen,
(b2) Reinigung der in Schritt (b1) erhaltenen Zusammensetzung (B1), um eine Mischung zu erhalten, die mindestens 92 Gew.-% der Verbindung mit Z-1 Cyclen und 7 bis 16 C-Atomen mit mindestens einer Aldehydgruppe und mindestens zwei C-C-Doppelbindungen enthält,
wobei Z 1, 2, 3 oder 4 sein kann, und Stufe (b1) in mindestens zwei Destillationskolonnen T1 und T2, wobei T1 mindestens 15 theoretische Trennstufen und T2 mindestens 30 Trennstufen aufweisen, oder in einer Trennwandkolonne, durchgeführt wird.

2. Verfahren nach Anspruch 1, **dadurch gekennzeichnet, dass** das in Stufe (a2) abgetrennte cyclische Olefin mit Z Cyclen und 7 bis 16 C-Atomen mit mindestens zwei C-C-Doppelbindungen wieder in Stufe (a1) zurückgeführt wird.

3. Verfahren nach Anspruch 1 oder 2, **dadurch gekennzeichnet, dass** die in Schritt (b1) abgetrennte cyclische Verbindung mit Z Cyclen und 7 bis 16 C-Atomen mit einer Ketogruppe mindestens eine C-C-Doppelbindung aufweist.

4. Verfahren nach Anspruch 3, **dadurch gekennzeichnet, dass** die cyclische Verbindung mit Z Cyclen und 7 bis 16 C-Atomen mit einer Ketogruppe mit mindestens einer C-C-Doppelbindung nach Schritt (b1) zu einer gesättigten cyclischen Verbindung mit Z Cyclen und 7 bis 16 C-Atomen mit einer Ketogruppe hydriert wird.

5. Verfahren nach Anspruch 1, **dadurch gekennzeichnet, dass** das cyclische Olefin 1,5,9-Cyclododecatrien ist, das durch Trimerisierung von Butadien hergestellt wurde.

6. Verfahren nach einem der Ansprüche 1 bis 5 **dadurch gekennzeichnet, dass** die Verbindung mit Z-1 Cyclen und 7 bis 16 C-Atomen mit mindestens einer Aldehydgruppe ein 4,8,11-Dodecatrienal ist.

7. Verwendung einer Mischung, enthaltend 92 bis 99,8 Gew.-% mindestens eines 4,8,11-Dodecatrienals und 0,0001 bis 5 Gew.-% 1,5,9-Cyclododecatriene, als Riechstoff.

8. Verwendung nach Anspruch 7, **dadurch gekennzeichnet, dass** sie 40 bis 50 Gew.-% (E,Z)-4,8,11-Dodecatrienal. 40 bis 50 Gew.-% (Z,E)-4,8,11-Dodecatrienal, 0,1 bis 10 Gew.-% (E,E)-4,8,11-Dodecatrienal und 0,0001 bis 5 Gew.-% 1,5,9-Cyclododecatriene enthält.

9. Verwendung nach Anspruch 7 oder 8 in einer Menge von 0,0001 bis 5 Gew.-%.

10. Verwendung nach Anspruch 7, 8 oder 9 in Parfüms, Kosmetika, Seifen, Reinigungsmitteln, Shampoos, Nahrungsmitteln, Hygieneprodukten oder Pharmazeutika.

## Claims

1. A process for preparing a compound with Z-1 cycles and 7 to 16 carbon atoms with at least one aldehyde group, comprising the stages:
(a1) oxidation of a cyclic olefin with Z cycles and 7 to 16 carbon atoms and at least two C-C double bonds, selected from the group consisting of 1,5-cyclooctadiene, 1,5-cyclododecadiene, 1,9-cyclohexadecadiene, 1,8-cyclotetradecadiene, 1,6-cyclodecadiene, 1,6,11-cyclopentadecatriene, 1,5,9-cyclododecatriene, vinylcyclohexene, norbornadiene, ethylidenenorbornene by means of dinitrogen monoxide to give a composition (A1), at least comprising
- a cyclic compound with Z cycles and 7 to 16 carbon atoms with a keto group,
- a cyclic olefin with Z cycles and 7 to 16 carbon atoms with at least two C-C double bonds and
- a compound with Z-1 cycles and 7 to 16 carbon atoms with at least one aldehyde group and at least two C-C double bonds,
(a2) separating off the cyclic olefin with Z cycles and 7 to 16 carbon atoms with at least two C-C double bonds from the composition (A1) from stage (a1) in order to obtain a composition (A2), at least comprising
- the cyclic compound with Z cycles and 7 to 16 carbon atoms with a keto group and
- the compound with Z-1 cycles and 7 to 16 carbon atoms with at least one aldehyde group and at least two C-C double bonds,
(b1) separating off the cyclic compound with Z cycles and 7 to 16 carbon atoms with a keto group from the composition (A2) from step (a2), in order to obtain a composition (B1), comprising at least 50% by weight of the compound with Z-1 cycles and 7 to 16 carbon atoms with at least one aldehyde group and at least two C-C double bonds,
(b2) purification of the composition (B1) obtained in step (b1) in order to obtain a mixture which comprises at least 92% by weight of the compound with Z-1 cycles and 7 to 16 carbon atoms with at least one aldehyde group and at least two C-C double bonds,
where Z may be 1, 2, 3 or 4, and stage (b1) is conducted in at least two distillation columns T1 and T2, where T1 has at least 15 theoretical plates and T2 at least 30 theoretical plates, or in a dividing wall column.

2. The process according to claim 1, wherein the cyclic olefin with Z cycles and 7 to 16 carbon atoms with at least two C-C double bonds separated off in stage (a2) is recycled again to stage (a1).

3. The process according to claim 1 or 2, wherein the cyclic compound with Z cycles and 7 to 16 carbon atoms with a keto group separated off in step (b1) has at least one C-C double bond.

4. The process according to claim 3, wherein the cyclic compound with Z cycles and 7 to 16 carbon atoms with a keto group with at least one C-C double bond is hydrogenated after step (b1) to give a saturated cyclic compound with Z cycles and 7 to 16 carbon atoms, with a keto group.

5. The process according to claim 1, wherein the cyclic olefin is 1,5,9-cyclododecatriene which has been prepared from butadiene by means of trimerization.

6. The process according to any one of claims 1 to 5, wherein the compound with Z-1 cycles and 7 to 16 carbon atoms with at least one aldehyde group is 4,8,11-dodecatrienal.

7. The use of a mixture comprising 92 to 99.8% by weight of at least one 4,8,11-dodecatrienal and 0.0001 to 5% by weight of 1,5,9-cyclododecatrienes as fragrance.

8. The use according to claim 7, which comprises 40 to 50% by weight of (E,Z)-4,8,11-dodecatrienal, 40 to 50% by weight of (Z,E)-4,8,11-dodecatrienal, 0.1 to 10% by weight of (E,E)-4,8,11-dodecatrienal and 0.0001 to 5% by weight of 1,5,9-cyclododecatrienes.

9. The use according to claim 7 or 8 in an amount of 0.0001 to 5% by weight.

10. The use according to claim 7, 8 or 9 in perfumes, cosmetics, soaps, cleaning compositions, shampoos, foods, hygiene products or pharmaceuticals.

## Revendications

1. Procédé de fabrication d'un composé à Z-1 cycles et 7 à 16 atomes C contenant au moins un groupe aldéhyde, comprenant les étapes suivantes :
(a1) l'oxydation d'une oléfine cyclique à Z cycles et 7 à 16 atomes C et contenant au moins deux doubles liaisons C-C, choisie dans le groupe constitué par le 1,5-cyclo-octadiène, le 1,5-cyclododécadiène, le 1,9-cyclo-hexadécadiène, le 1,8-cyclotétradécadiène, le 1,6-cyclo-décadiène, le 1,6,11-cyclopentadécatriène, le 1,5,9-cyclododécatriène, le vinylcyclohexène, le norbornadiène, l'éthylidène-norbornène, au moyen de monoxyde de diazote pour obtenir une composition (A1) contenant au moins :
- un composé cyclique à Z cycles et 7 à 16 atomes C contenant un groupe céto,
- une oléfine cyclique à Z cycles et 7 à 16 atomes C contenant au moins deux doubles liaisons C-C, et
- un composé à Z-1 cycles et 7 à 16 atomes C contenant au moins un groupe aldéhyde et au moins deux doubles liaisons C-C,
(a2) la séparation de l'oléfine cyclique à Z cycles et 7 à 16 atomes C contenant au moins deux doubles liaisons C-C de la composition (A1) de l'étape (a1) pour obtenir une composition (A2), contenant au moins :
- le composé cyclique à Z cycles et 7 à 16 atomes C contenant un groupe céto, et
- le composé à Z-1 cycles et 7 à 16 atomes C contenant au moins un groupe aldéhyde et au moins deux doubles liaisons C-C,
(b1) la séparation du composé cyclique à Z cycles et 7 à 16 atomes C contenant un groupe céto de la composition (A2) de l'étape (a2) pour obtenir une composition (B1), contenant au moins 50 % en poids du composé à Z-1 cycles et 7 à 16 atomes C contenant au moins un groupe aldéhyde et au moins deux doubles liaisons C-C,
(b2) la purification de la composition (B1) obtenue à l'étape (b1) pour obtenir un mélange qui contient au moins 92 % en poids du composé à Z-1 cycles et 7 à 16 atomes C contenant au moins un groupe aldéhyde et au moins deux doubles liaisons C-C,
Z pouvant être 1, 2, 3 ou 4, et l'étape (b1) étant réalisée dans au moins deux colonnes de distillation T1 et T2, T1 comprenant au moins 15 étapes de séparation théoriques et T2 au moins 30 étapes de séparation, ou dans une colonne à paroi de séparation.

2. Procédé selon la revendication 1, **caractérisé en ce que** l'oléfine cyclique à Z cycles et 7 à 16 atomes C contenant au moins deux doubles liaisons C-C séparée à l'étape (a2) est réintroduite dans l'étape (a1).

3. Procédé selon la revendication 1 ou 2, **caractérisé en ce que** le composé cyclique à Z cycles et 7 à 16 atomes C contenant un groupe céto séparé à l'étape (b1) comprend au moins une double liaison C-C.

4. Procédé selon la revendication 3, **caractérisé en ce que** le composé cyclique à Z cycles et 7 à 16 atomes C contenant un groupe céto qui contient au moins une double liaison C-C est hydrogéné après l'étape (b1) en un composé cyclique saturé à Z cycles et 7 à 16 atomes C contenant un groupe céto.

5. Procédé selon la revendication 1, **caractérisé en ce que** l'oléfine cyclique est le 1,5,9-cyclododécatriène, qui a été fabriqué par trimérisation de butadiène.

6. Procédé selon l'une quelconque des revendications 1 à 5, **caractérisé en ce que** le composé à Z-1 cycles et 7 à 16 atomes C contenant au moins un groupe aldéhyde est un 4,8,11-dodécatriénal.

7. Utilisation d'un mélange contenant 92 à 99,8 % en poids d'au moins un 4,8,11-dodécatriénal et 0,0001 à 5 % en poids de 1,5,9-cyclododécatriène en tant que substance odoriférante.

8. Utilisation selon la revendication 7, **caractérisée en ce qu'**il contient 40 à 50 % en poids d'(E,Z)-4,8,11-dodécatriénal, 40 à 50 % en poids de (Z,E)-4,8,11-dodécatriénal, 0,1 à 10 % en poids d'(E,E)-4,8,11-dodécatriénal et 0,0001 à 5 % en poids de 1,5,9-cyclododécatriène.

9. Utilisation selon la revendication 7 ou 8 en une quantité de 0,0001 à 5 % en poids.

10. Utilisation selon la revendication 7, 8 ou 9 dans des parfums, des produits cosmétiques, des savons, des détergents, des shampoings, des produits alimentaires, des produits d'hygiène ou des produits pharmaceutiques.
